(19) Europäisches Patentamt / European Patent Office / Office européen des brevets

(11) **EP 4 082 571 A1**

(12) **EUROPEAN PATENT APPLICATION**
published in accordance with Art. 153(4) EPC

(43) Date of publication:
**02.11.2022 Bulletin 2022/44**

(21) Application number: **20907782.5**

(22) Date of filing: **24.12.2020**

(51) International Patent Classification (IPC):
***A61K 39/395*** (2006.01) ***A61K 45/06*** (2006.01)
***A61P 35/00*** (2006.01) ***A61P 43/00*** (2006.01)
***A61K 31/4184*** (2006.01)

(52) Cooperative Patent Classification (CPC):
**A61K 31/4184; A61K 39/395; A61K 45/06; A61P 35/00; A61P 43/00**

(86) International application number:
**PCT/JP2020/048480**

(87) International publication number:
**WO 2021/132473 (01.07.2021 Gazette 2021/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.12.2019 JP 2019233866**

(71) Applicant: **Nippon Shinyaku Co., Ltd.**
**Kyoto-shi**
**Kyoto 601-8550 (JP)**

(72) Inventors:
- **KOSUGI Keiji**
  **Kyoto-shi, Kyoto 601-8550 (JP)**
- **MINAMI Toshiyuki**
  **Kyoto-shi, Kyoto 601-8550 (JP)**
- **IWASAKI Shiho**
  **Kyoto-shi, Kyoto 601-8550 (JP)**
- **YAMAMOTO Itaru**
  **Kyoto-shi, Kyoto 601-8550 (JP)**

(74) Representative: **Hoffmann Eitle**
**Patent- und Rechtsanwälte PartmbB**
**Arabellastraße 30**
**81925 München (DE)**

# (54) ANTITUMOR DRUG FOR USE IN COMBINATION WITH IMMUNE CHECKPOINT INHIBITOR

(57) The present invention relates to a prophylactic and/or therapeutic agent for cancer containing an mPGES-1 inhibitor as an active ingredient, the agent being useful in combination with an immune checkpoint inhibitor, and the invention has industrial applicability.

Fig. 1

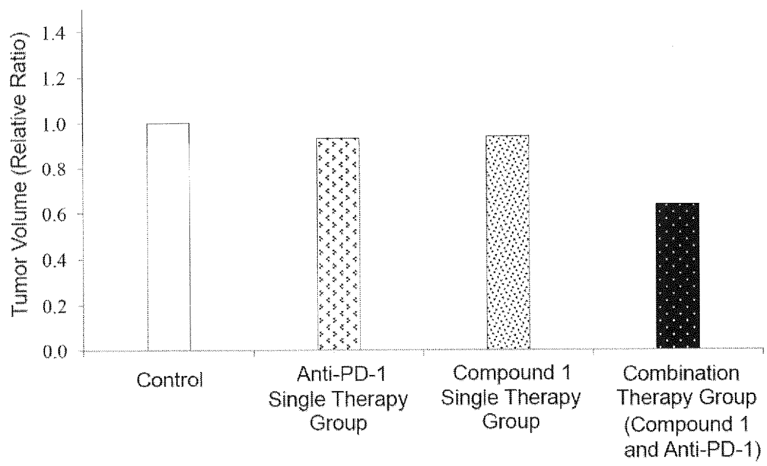


EP 4 082 571 A1

## Description

TECHNICAL FIELD

The present invention relates to a prophylactic and/or therapeutic agent for cancer containing an mPGES-1 inhibitor as an active ingredient, which is useful in combination with an immune checkpoint inhibitor, and a prophylactic and/or therapeutic agent for cancer containing an mPGES-1 inhibitor and an immune checkpoint inhibitor as active ingredients.

BACKGROUND ART

With regard to PGE2 synthase (PGES), it is known that there are three sub-types such as membrane-bound prostaglandin E synthase-1 (mPGES-1), mPGES-2, and cytoplasmic PGES (cPGES) (Non-Patent Literatures 1 to 3). mPGES-1, in the same manner as COX-2, is primarily induced during inflammation and plays a major part in PGE2 production in inflammatory lesions. Furthermore, it is known that mPGES-1 is involved in malignant tumors (for example, colon cancer, breast cancer, lung cancer, and prostate cancer) (see Non-Patent Literature 1, Non-Patent Literature 2, and Non-Patent Literature 3).
It is described in Patent Literature 1 that a heterocyclic derivative represented by General Formula [1] or a tautomer of the derivative or a pharmaceutically acceptable salt thereof has mPGES-1 inhibitory activity.
In Non-Patent Literature 4, it is described that immune checkpoint inhibitors are used in combination, or an immune checkpoint inhibitor and another anticancer agent are used in combination.
However, nothing has been reported so far on the prevention and/or treatment of cancer by using an mPGES-1 inhibitor and an immune checkpoint inhibitor in combination.

CITATION LIST

PATENT LITERATURE

Patent Literature 1: WO 2013/024898

NON-PATENT LITERATURE

Non-Patent Literature 1: J. Biol. Chem., 2003, 278(21), 19396-19405.
Non-Patent Literature 2: Oncogene, 2012, 31(24), 2943-2952.
Non-Patent Literature 3: Cancer Res., 2008, 68(9), 3251-3259.
Non-Patent Literature 4: Front Immunol. 2018 Jul 27;9: 1739.

SUMMARY OF INVENTION

TECHNICAL PROBLEM

An object to be achieved by the present invention is to provide a novel prophylactic and/or therapeutic agent for cancer.

SOLUTION TO PROBLEM

The inventors of the present invention found that antitumor activity is synergistically enhanced by using an mPGES-1 inhibitor and an immune checkpoint inhibitor in combination, thus completing the invention.
The present invention relates to the following.

(1) A prophylactic and/or therapeutic agent for cancer containing an mPGES-1 inhibitor as an active ingredient, for use in combination with an immune checkpoint inhibitor.
(2) The prophylactic and/or therapeutic agent for cancer according to (1),

wherein the mPGES-1 inhibitor is a compound of Formula [1] (hereinafter, also referred to as present compound), a tautomer of the compound, or a pharmaceutically acceptable salt thereof, and

the compound of Formula [1] is as follow:

Formula [1]:

[Chemical Formula 1]

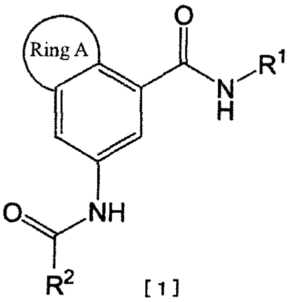

[1]

wherein
ring A represents a group represented by Formula [2], [3], or [4]:

[Chemical Formula 2]

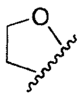

[2]

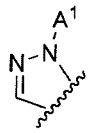

[3]

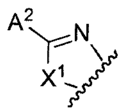

[4]

wherein

$X^1$ represents NH, N-alkyl, or O;
$A^1$ represents hydrogen or alkyl;
$A^2$ represents:

i) hydrogen,
ii) halogen,
iii) alkyl which may be substituted with one to three groups selected from the group consisting of halogen, amino, monoalkylamino, dialkylamino, carbamoyl, monoalkylaminocarbonyl, dialkylaminocarbonyl, saturated cyclic aminocarbonyl, alkoxy, alkoxyalkoxy, and alkylcarbonyloxy,
iv) cycloalkyl which may be substituted with alkyl, wherein said alkyl may be substituted with one to three halogens,
v) alkoxy,
vi) a saturated heterocyclic group which may be substituted with alkyl, alkyloxycarbonyl, alkylcarbonyl, or oxo,
vii) alkylthio,
viii) alkylsulfonyl,
ix) alkylsulfinyl,
x) Formula [5]:

[Chemical Formula 3]

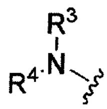

[5]

wherein $R^3$ and $R^4$ are identical or different and each represent:

a) hydrogen,
b) alkyl which may be substituted with a group selected from the group consisting of monoalkylamino, dialkylamino, saturated cyclic amino optionally substituted with alkyl, a saturated heterocyclic group optionally substituted with alkyl, alkoxy, hydroxycarbonyl, hydroxyl, alkyloxycarbonyl, and alkylthio, or
c) cycloalkyl,
or

xi) saturated cyclic amino which may be substituted with alkyl, amino, monoalkylamino, dialkylamino, alkoxy, or hydroxyl;

$R^1$ represents phenyl, benzyl, naphthyl, cycloalkyl, cycloalkylmethyl, heteroaryl, heteroarylmethyl, 1,2,3,4-tetrahydronaphthalen-5-yl, 1,2,3,4-tetrahydronaphthalen-6-yl, 2,3-dihydro-1H-inden-4-yl, 2,3-dihydro-1H-inden-5-yl, 1,2-dihydrocyclobutabenzen-3-yl, 1,2-dihydrocyclobutabenzen-4-yl, or alkyl, wherein said phenyl, benzyl, cycloalkyl, cycloalkylmethyl, heteroaryl, and heteroarylmethyl may be substituted with one to three groups selected from the group consisting of:

i) halogen,
ii) alkyl which may be substituted with one to three groups selected from the group consisting of halogen, hydroxy, and phenyl,
iii) alkoxy,
iv) hydroxy, and
v) cyano;

$R^2$ represents phenyl or pyridyl, wherein said phenyl and pyridyl may be substituted with one to three groups selected from the group consisting of:

i) halogen,
ii) alkylsulfonyl,
iii) alkoxy which may be substituted with one to three halogens or alkoxies,
iv) alkynyl which may be substituted with alkoxyalkyl or cycloalkyl, and
v) alkyl which may be substituted with one to three groups selected from the group consisting of alkoxy, alkoxyalkoxy, cycloalkyl, phenyl, and halogen.

(3) The prophylactic and/or therapeutic agent for cancer according to (2), wherein the ring A represents a group represented by Formula [4], and $X^1$ represents NH.
(4) The prophylactic and/or therapeutic agent for cancer according to (2) or (3),
wherein $R^1$ represents phenyl, 1,2,3,4-tetrahydronaphthalen-5-yl, 1,2,3,4-tetrahydronaphthalen-6-yl, 2,3-dihydro-1H-inden-4-yl, 2,3-dihydro-1H-inden-5-yl, 1,2-dihydrocyclobutabenzen-3-yl, or 1,2-dihydrocyclobutabenzen-4-yl, wherein said phenyl may be substituted with one to three groups selected from the group consisting of:

i) halogen,
ii) alkyl which may be substituted with one to three halogens,
iii) alkoxy, and
iv) cyano.

(5) The prophylactic and/or therapeutic agent for cancer according to any one of (2) to (4),

wherein $R^2$ represents phenyl, and
said phenyl may be substituted with one to three groups selected from the group consisting of:

i) halogen,
ii) alkylsulfonyl,
iii) alkoxy which may be substituted with alkoxy,
iv) alkynyl which may be substituted with alkoxyalkyl or cycloalkyl, and
v) alkyl which may be substituted with one to three groups selected from the group consisting of halogen,

alkoxy, alkoxyalkoxy, cycloalkyl, and phenyl.

(6) The prophylactic and/or therapeutic agent for cancer according to any one of (2) to (5),

wherein the ring A represents a group represented by General Formula [4];
$X^1$ represents NH;
$A^2$ represents:

i) hydrogen,
ii) alkyl which may be substituted with a group selected from the group consisting of halogen, monoalkylamino, dialkylamino, monoalkylaminocarbonyl, dialkylaminocarbonyl, saturated cyclic aminocarbonyl, alkoxy, alkoxyalkoxy, and alkylcarbonyloxy,
iii) cycloalkyl which may be substituted with alkyl optionally substituted with one to three halogens,
iv) alkoxy,
v) a saturated heterocyclic group which may be substituted with alkyl or alkyloxycarbonyl,
vi) alkylthio,
vii) alkylsulfonyl,
viii) alkylsulfinyl,
ix) amino substituted with alkyl which may be substituted with a group selected from the group consisting of monoalkylamino, dialkylamino, saturated cyclic amino optionally substituted with alkyl, tetrahydrofuryl, morpholino, alkoxy, hydroxycarbonyl, hydroxyl, and alkylthio,
x) amino substituted with cycloalkyl, or
xi) saturated cyclic amino which may be substituted with alkyl, dialkylamino, alkoxy, or hydroxyl;

$R^1$ represents:

i) phenyl which may be substituted with one to three groups selected from the group consisting of halogen, alkyl optionally substituted with one to three halogens, alkoxy, and cyano,
ii) 1,2,3,4-tetrahydronaphthalen-5-yl,
iii) 2,3-dihydro-1H-inden-5-yl,
iv) benzyl which may be substituted with halogen or with alkyl optionally substituted with one to three halogens,
v) cycloalkyl,
vi) cycloalkylmethyl,
vii) naphthyl,
viii) pyridylmethyl which may be substituted with alkyl optionally substituted with one to three halogens,
ix) thienyl,
x) thienylmethyl,
xi) benzothiazolyl,
xii) benzothiadiazolyl,
xiii) indolyl, or
xiv) alkyl; and

$R^2$ represents phenyl or pyridyl,

wherein said phenyl may be substituted with one to three groups selected from the group consisting of:

i) halogen,
ii) alkylsulfonyl,
iii) alkoxy which may be substituted with alkoxy,
iv) alkynyl which may be substituted with alkoxyalkyl or cycloalkyl, and
v) alkyl which may be substituted with one to three groups selected from the group consisting of halogen, alkoxy, alkoxyalkoxy, cycloalkyl, and phenyl, and

said pyridyl may be substituted with halogen.

(7) The prophylactic and/or therapeutic agent for cancer according to any one of (2) to (6),

wherein the ring A represents a group represented by General Formula [4];
$X^1$ represents NH;
$A^2$ represents alkoxy-substituted alkyl, dialkylamino, tetrahydrofuryl, tetrahydrofurylmethyl, alkoxyalkylamino, or cycloalkyl which may be substituted with unsubstituted alkyl or alkyl substituted with one to three halogens;
$R^1$ represents phenyl substituted with one halogen and one methyl; and
$R^2$ represents phenyl which may be substituted with one trifluoromethyl or two halogens.

(8) The prophylactic and/or therapeutic agent for cancer according to any one of (2) to (7), wherein the mPGES-1 inhibitor is a compound selected from the group consisting of:

(1) N-[2-(trifluoromethyl)benzyl]-6-({[2-(trifluoromethyl)phenyl]carbonyl}amino)-1H-benzimidazole-4-carboxamide,
(2) N-cyclohexyl-6-({[2-(trifluoromethyl)phenyl]carbonyl}amino)-1H-benzimidazole-4-carboxamide,
(3) N-(3-chloro-2-methylphenyl)-6-({[2-(trifluoromethyl)phenyl]carbonyl} amino)-1H-benzimidazole-4-carboxamide,
(4) N-[(1-hydroxycyclohexyl)methyl]-6-({[2-(trifluoromethyl)phenyl]carbonyl}amino)-1H-benzimidazole-4-carboxamide,
(5) N-[2-(trifluoromethyl)benzyl]-5-({[2-(trifluoromethyl)phenyl]carbonyl}amino} - 2,3-dihydro-1-benzofuran-7-carboxamide,
(6) N-cyclohexyl-5-({[2-(trifluoromethyl)phenyl]carbonyl}amino)-2,3-dihydro-1-benzofuran-7-carboxamide,
(7) N-(3-chloro-2-methylphenyl)-5-({[2-(trifluoromethyl)phenyl]carbonyl} amino)-2,3-dihydro-1-benzofuran-7-carboxamide,
(8) N-cyclohexyl-5-({[2-(trifluoromethyl)phenyl]carbonyl}amino)-1H-indazole-7-carboxamide,
(9) N-[2-(trifluoromethyl)benzyl]-5-({[2-(trifluoromethyl)phenyl]carbonyl}amino)-1H-indazole-7-carboxamide,
(10) N-(3-chloro-2-methylphenyl)-2-(methoxymethyl)-6-({[2-(trifluoromethyl)phenyl] carbonyl }amino)-1H-benzimidazole-4-carboxamide,
(11) 2-methyl-N-[2-(trifluoromethyl)benzyl]-6-({[2-(trifluoromethyl)phenyl]carbonyl}amino)-1H-benzimidazole-4-carboxamide,
(12) N-cyclohexyl-2-methyl-6-({[2-(trifluoromethyl)phenyl]carbonyl}amino)-1H-benzimidazole-4-carboxamide,
(13) N-(3-chloro-2-methylphenyl)-2-methyl-6-({[2-(trifluoromethyl)phenyl]carbonyl}amino)-1H-benzimidazole-4-carboxaniide,
(14) N-cyclopentyl-2-methyl-6-({[2-(trifluoromethyl)phenyl] carbonyl}amino)-1H-benzimidazole-4-carboxamide,
(15) N-cyclobutyl-2-methyl-6-({[2-(trifluoromethyl)phenyl]carbonyl}amino)-1H-benzimidazole-4-carboxamide,
(16) N-(3-chloro-2-methylphenyl)-2-ethyl-6-({[2-(trifluoromethyl)phenyl]carbonyl}amino)-1H-benzimidazole-4-carboxamide,
(17) N-cyclohexyl-2-ethyl-6-({[2-(trifluoromethyl)phenyl]carbonyl}amino)-1H-benzimidazole-4-carboxamide,
(18) 2-ethyl-N-[2-(trifluoromethyl)benzyl]-6-({[2-(trifluoromethyl)phenyl]carbonyl}amino)-1H-benzimidazole-4-carboxamide,
(19) N-cyclohexyl-2-(methoxymethyl)-6-({[2-(trifluoromethyl)phenyl]carbonyl}amino)-1H-benzimidazole-4-carboxamide,
(20) 2-(methoxymethyl)-N-[2-(trifluoromethyl)benzyl]-6-({[2-(trifluoromethyl)phenyl]carbonyl}amino)-1H-benzimidazole-4-carboxamide,
(21) 2-(methoxymethyl)-N-(2-methylphenyl)-6-({[2-(trifluoromethyl)phenyl]carbonyl}amino)-1H-benzimidazole-4-carboxamide,
(22) 2-(methoxymethyl)-N-(4-methylphenyl)-6-({[2-(trifluoromethyl)phenyl]carbonyl}amino)-1H-benzimidazole-4-carboxamide,
(23) N-(2-chlorobenzyl)-2-(methoxymethyl)-6-({[2-(trifluoromethyl)phenyl]carbonyl}amino)-1H-benzimidazole-4-carboxamide,
(24) 2-(methoxymethyl)-N-(4-methylbenzyl)-6-({[2-(trifluoromethyl)phenyl]carbonyl}amino)-1H-benzimidazole-4-carboxamide,
(25) N-(4,4-difluorocyclohexyl)-2-(methoxymethyl)-6-({[2-(trifluoromethyl)phenyl]carbonyl} amino)-1H-benzimidazole-4-carboxamide,
(26) N-(4-tert-butylphenyl)-2-(methoxymethyl)-6-({[2-(trifluoromethyl)phenyl]carbonyl}amino)-1H-benzimidazole-4-carboxamide,
(27) 2-(methoxymethyl)-N-[4-(trifluoromethyl)phenyl]-6-({[2-(trifluoromethyl)phenyl] carbonyl} amino)-1H-benzimidazole-4-carboxamide,
(28) N-(2,4-dimethylphenyl)-2-(methoxymethyl)-6-({[2-(trifluoromethyl)phenyl]carbonyl}amino)-1H-benzimidazole-4-carboxamide,

(29) N-(2-chloro-4-methylphenyl)-2-(methoxymethyl)-6-({[2-(trifluoromethyl)phenyl]carbonyl}amino)-1H-benzimidazole-4-carboxamide,
(30) N-(3,4-dimethylphenyl)-2-(methoxymethyl)-6-({[2-(trifluoromethyl)phenyl]carbonyl}amino)-1H-benzimidazole-4-carboxamide,
(31) N-(3-chloro-4-methylphenyl)-2-(methoxymethyl)-6-({[2-(trifluoromethyl)phenyl]carbonyl}amino)-1H-benzimidazole-4-carboxamide,
(32) N-(2,3-dihydro-1H-inden-5-yl)-2-(methoxymethyl)-6-({[2-(trifluoromethyl)phenyl]carbonyl}amino)-1H-benzimidazole-4-carboxamide,
(33) 2-(methoxymethyl)-N-(5,6,7,8-tetrahydronaphthalen-1-yl)-6-({[2-(trifluoromethyl)phenyl]carbonyl}amino)-1H-benzimidazole-4-carboxamide,
(34) N-(2-fluorophenyl)-2-(methoxymethyl)-6-({[2-(trifluoromethyl)phenyl]carbonyl}amino)-1H-benzimidazole-4-carboxamide,
(35)2-(methoxymethyl)-N-(2-methoxyphenyl)-6-({[2-(trifluoromethyl)phenyl] carbonyl}amino)-1H-benzimidazole-4-carboxamide,
(36) 2-(methoxymethyl)-N-(4-methoxyphenyl)-6-({[2-(trifluoromethyl)phenyl]carbonyl}amino)-1H-benzimidazole-4-carboxamide,
(37) N-(3-bromo-2-methylphenyl)-2-(methoxymethyl)-6-({[2-(trifluoromethyl)phenyl]carbonyl}amino)-1H-benzimidazole-4-carboxamide,
(38) N-(3-chloro-2-methylbenzyl)-2-(methoxymethyl)-6-({[2-(trifluoromethyl)phenyl]carbonyl}amino)-1H-benzimidazole-4-carboxamide,
(39) N-(2,6-difluorophenyl)-2-(methoxymethyl)-6-({[2-(trifluoromethyl)phenyl]carbonyl}amino)-1H-benzimidazole-4-carboxamide,
(40) N-(3-cyano-2-methylphenyl)-2-(methoxymethyl)-6-({[2-(trifluoromethyl)phenyl]carbonyl}amino)-1H-benzimidazole-4-carboxamide,
(41) 2-(methoxymethyl)-6-({[2-(trifluoromethyl)phenyl]carbonyl} amino)-N-{[3-(trifluoromethyl)pyridin-2-yl]methyl}-1H-benzimidazole-4-carboxamide,
(42) N-(2-chloro-6-methylphenyl)-2-(methoxymethyl)-6-({[2-(trifluoromethyl)phenyl]carbonyl}amino)-1H-benzimidazole-4-carboxamide,
(43) 2-(2-amino-2-oxoethyl)-N-(3-chloro-2-methylphenyl)-6-({[2-(trifluoromethyl)phenyl]carbonyl}amino)-1H-benzimidazole-4-carboxamide,
(44) 2-(2-amino-2-oxoethyl)-N-[2-(trifluoromethyl)benzyl]-6-({[2-(trifluoromethyl)phenyl]carbonyl} amino)-1H-benzimidazole-4-carboxamide,
(45) N-(3-chloro-2-methylphenyl)-1-methyl-6-({[2-(trifluoromethyl)phenyl]carbonyl}amino)-1H-benzimidazole-4-carboxamide,
(46) N-cyclohexyl-1-methyl-6-({[2-(trifluoromethyl)phenyl]carbonyl}amino)-1H-benzimidazole-4-carboxamide,
(47) 1-methyl-N-[2-(trifluoromethyl)benzyl]-6-({[2-(trifluoromethyl)phenyl]carbonyl}amino)-1H-benzimidazole-4-carboxamide,
(48) N-(3-chloro-2-methylphenyl)-1-ethyl-6-({[2-(trifluoromethyl)phenyl]carbonyl}amino)-1H-benzimidazole-4-carboxamide,
(49) N-cyclohexyl-1-ethyl-6-({[2-(trifluoromethyl)phenyl]carbonyl} amino)-1H-benzimidazole-4-carboxamide,
(50) 1-ethyl-N-[2-(trifluoromethyl)benzyl]-6-({[2-(trifluoromethyl)phenyl]carbonyl}amino)-1H-benzimidazole-4-carboxamide,
(51) N-(3-chloro-2-methylphenyl)-2-methyl-6-({[2-(trifluoromethyl)phenyl] carbonyl} amino)-1 ,3-benzoxazole-4-carboxamide,
(52) 2-methyl-N-[2-(trifluoromethyl)benzyl]-6-({[2-(trifluoromethyl)phenyl]carbonyl}amino)-1,3-benzoxazole-4-carboxamide,
(53) N-(3-chloro-2-methylphenyl)-2-ethyl-6-({[2-(trifluoromethyl)phenyl]carbonyl}amino)-1,3-benzoxazole-4-carboxamide,
(54) N-(3-chloro-2-methylphenyl)-2-ethoxy-6-({[2-(trifluoromethyl)phenyl]carbonyl}amino)-1H-benzimidazole-4-carboxamide,
(55) 2-ethoxy-N-[2-(trifluoromethyl)benzyl]-6-({[2-(trifluoromethyl)phenyl]carbonyl}amino)-1H-benzimidazole-4-carboxamide,
(56) N-(3-chloro-2-methylphenyl)-2-(1-chloro-2-methylpropan-2-yl)-6-({[2-(trifluoromethyl)phenyl] carbonyl}amino)-1H-benzimidazole-4-carboxamide,
(57) N-(3-chloro-2-methylphenyl)-2-[(dimethylamino)methyl]-6-({[2-(trifluoromethyl)phenyl]carbonyl}amino)-1H-benzimidazole-4-carboxamide,
(58) N-(3-chloro-2-methylphenyl)-2-(2-methylpropyl)-6-({[2-(trifluoromethyl)phenyl]carbonyl}amino)-1H-benzimidazole-4-carboxamide,

(59) 2-(2-methylpropyl)-N-[2-(trifluoromethyl)benzyl]-6-({[2-(trifluoromethyl)phenyl]carbonyl}amino)-1H-benzimidazole-4-carboxamide,
(60) tert-butyl 3-{4-[(3-chloro-2-methylphenyl)carbamoyl]-6-({[2-(trifluoromethyl)phenyl]carbonyl}amino)-1H-benzimidazol-2-yl}azetidine-1-carboxylate,
(61) N-(3-chloro-2-methylphenyl)-2-[(methylamino)methyl]-6-({[2-(trifluoromethyl)phenyl]carbonyl}amino)-1H-benzimidazole-4-carboxamide,
(62) {4-[(3-chloro-2-methylphenyl)carbamoyl]-6-({[2-(trifluoromethyl)phenyl]carbonyl}amino)-1H-benzimidazol-2-yl}methyl acetate,
(63) N-(3-chloro-2-methylphenyl)-2-[(2R)-tetrahydrofuran-2-yl]-6-({[2-(trifluoromethyl)phenyl]carbonyl}amino)-1H-benzimidazole-4-carboxamide,
(64) 2-[(2R)-tetrahydrofuran-2-yl]-N-[2-(trifluoromethyl)benzyl]-6-({[2-(trifluoromethyl)phenyl] carbonyl}amino)-1H-benzimidazole-4-carboxamide,
(65) N-(3-chloro-2-methylphenyl)-2-[(2S)-tetrahydrofuran-2-yl]-6-({[2-(trifluoromethyl)phenyl]carbonyl}amino)-1H-benzimidazole-4-carboxamide,
(66) 2-[(2S)-tetrahydrofuran-2-yl]-N-[2-(trifluoromethyl)benzyl]-6-({[2-(trifluoromethyl)phenyl]carbonyl}amino)-1H-benzimidazole-4-carboxamide,
(67) 2-(1-acetylazetidin-3-yl)-N-(3-chloro-2-methylphenyl)-6-({[2-(trifluoromethyl)phenyl]carbonyl} amino)-1H-benzimidazole-4-carboxamide,
(68) tert-butyl (2S)-2- {4-[(3-chloro-2-methylphenyl)carbamoyl]-6-({[2-(trifluoromethyl)phenyl]carbonyl}amino)-1H-benzimidazol-2-yl}pyrrolidine-1-carboxylate,
(69) tert-butyl (2R)-2-{4-[(3-chloro-2-methylphenyl)carbamoyl]-6-({[2-(trifluoromethyl)phenyl]carbonyl}amino)-1H-benzimidazol-2-yl}pyrrolidine-1-carboxylate,
(70) N-(3-chloro-2-methylphenyl)-2-[(2S)-pyrrolidin-2-yl]-6-({[2-(trifluoromethyl)phenyl]carbonyl}amino)-1H-benzimidazole-4-carboxamide,
(71) N-(3-chloro-2-methylphenyl)-2-[(2S)-1-methylpyrrolidin-2-yl]-6-({[2-(trifluoromethyl)phenyl]carbonyl}amino)-1H-benzimidazole-4-carboxamide,
(72) 2-[(2S)-1-acetylpyrrolidin-2-yl]-N-(3-chloro-2-methylphenyl)-6-({[2-(trifluoromethyl)phenyl]carbonyl}amino)-1H-benzimidazole-4-carboxamide,
(73) N-(3-chloro-2-methylphenyl)-2-[(2-methoxyethoxy)methyl]-6-({[2-(trifluoromethyl)phenyl]carbonyl}amino)-1H-benzimidazole-4-carboxamide,
(74) N-(3-chloro-2-methylphenyl)-2-(1-methoxy-2-methylpropan-2-yl)-6-({[2-(trifluoromethyl)phenyl]carbonyl}amino)-1H-benzimidazole-4-carboxamide,
(75) 2-tert-butyl-N-(3-chloro-2-methylphenyl)-6-({[2-(trifluoromethyl)phenyl]carbonyl}amino)-1H-benzimidazole-4-carboxamide,
(76) 2-tert-butyl-6-({[2-(trifluoromethyl)phenyl]carbonyl}amino)-N-{[3-(trifluoromethyl)pyridin-2-yl]methyl}-1H-benzimidazole-4-carboxamide,
(77) N-(3-chloro-2-methylphenyl)-2-(2-ethoxyethyl)-6-({[2-(trifluoromethyl)phenyl]carbonyl}amino)-1H-benzimidazole-4-carboxamide,
(78) N-(3-chloro-2-methylphenyl)-2-(ethoxymethyl)-6-({[2-(trifluoromethyl)phenyl]carbonyl}amino)-1H-benzimidazole-4-carboxamide,
(79) 2-(ethoxymethyl)-6-({[2-(trifluoromethyl)phenyl]carbonyl}amino)-N-{[3-(trifluoromethyl)pyridin-2-yl]methyl}-1H-benzimidazole-4-carboxamide,
(80) N-(3-chloro-2-methylphenyl)-2-(2-methoxyethyl)-6-({[2-(trifluoromethyl)phenyl]carbonyl}amino)-1H-benzimidazole-4-carboxamide,
(81) N-(3-chloro-2-methylphenyl)-2-(2,2-dimethylpropyl)-6-({[2-(trifluoromethyl)phenyl]carbonyl}amino)-1H-benzimidazole-4-carboxamide,
(82) N-(3-chloro-2-methylphenyl)-2-cyclopropyl-6-({[2-(trifluoromethyl)phenyl]carbonyl}amino)-1H-benzimidazole-4-carboxamide,
(83) N-(3-chloro-2-methylphenyl)-2-(2-methylpentan-2-yl)-6-({[2-(trifluoromethyl)phenyl]carbonyl}amino)-1H-benzimidazole-4-carboxamide,
(84) N-(3-chloro-2-methylphenyl)-2-(1-methylcyclopropyl)-6-({[2-(trifluoromethyl)phenyl]carbonyl}amino)-1H-benzimidazole-4-carboxamide,
(85) 2-tert-butyl-N-(3-chloro-4-methylphenyl)-6-({[2-(trifluoromethyl)phenyl]carbonyl}amino)-1H-benzimidazole-4-carboxamide,
(86) 2-tert-butyl-N-(3-chloro-2-methylphenyl)-6-{[(2,5-dichlorophenyl)carbonyl]amino}-1H-benzimidazole-4-carboxamide,
(87) 2-tert-butyl-N-(3-chloro-4-methylphenyl)-6-{[(2,5-dichlorophenyl)carbonyl] amino}-1H-benzimidazole-4-carboxamide,

(88) N-(3-chloro-2-methylphenyl)-2-[1-(trifluoromethyl)cyclopropyl]-6-({[2-(trifluoromethyl)phenyl]carbonyl}amino)-1H-benzimidazole-4-carboxamide,
(89) N-(3-chloro-2-methylphenyl)-2-(methoxymethyl)-1-methyl-6-({[2-(trifluoromethyl)phenyl]carbonyl}amino)-1H-benzimidazole-4-carboxamide,
(90) N-(2-chlorobenzyl)-2-(methoxymethyl)-1-methyl-6-({[2-(trifluoromethyl)phenyl]carbonyl}amino)-1H-benzimidazole-4-carboxamide,
(91) 6- {[(2-chloro-6-fluorophenyl)carbonyl]amino} -N-(3-chloro-2-methylphenyl)-2-(methoxymethyl)-1H-benzimidazole-4-carboxamide,
(92) 6-{[(2-chloro-4-fluorophenyl)carbonyl]amino}-N-(3-chloro-2-methylphenyl)-2-methoxymethyl-1H-benzimidazole-4-carboxamide,
(93) 6-{[(2-chloro-5-fluorophenyl)carbonyl]amino}-N-(3-chloro-2-methylphenyl)-2-(methoxymethyl)-1H-benzimidazole-4-carboxamide,
(94) N-(3-chloro-2-methylphenyl)-6-{[(2-chlorophenyl)carbonyl]amino} -2-(methoxymethyl)-1H-benzimidazole-4-carboxamide,
(95) N-(3-chloro-2-methylphenyl)-6-{[(2-chloropyridin-3-yl)carbonyl]amino}-2-(methoxymethyl)-1H-benzimidazole-4-carboxamide,
(96) 6-{[(2-bromophenyl)carbonyl] amino} -N-(3-chloro-2-methylphenyl)-2-(methoxymethyl)-1H-benzimidazole-4-carboxamide,
(97) N-(3-chloro-2-methylphenyl)-6-{[(2,6-dichlorophenyl)carbonyl]amino}-2-(methoxymethyl)-1H-benzimidazole-4-carboxamide,
(98) N-(3-chloro-2-methylphenyl)-6- {[(2,5-dichlorophenyl)carbonyl]amino} -2-(methoxymethyl)-1H-benzimidazole-4-carboxamide,
(99) 6-{[(2-chloro-3-fluorophenyl)carbonyl]amino}-N-(3-chloro-2-methylphenyl)-2-(methoxymethyl)-1H-benzimidazole-4-carboxamide,
(100) 6-{[(2-chloro-3,6-difluorophenyl)carbonyl]amino}-N-(3-chloro-2-methylphenyl)-2-(methoxymethyl)-1H-benzimidazole-4-carboxamide,
(101) 6-{[(2-bromo-6-chlorophenyl)carbonyl]amino}-N-(3-chloro-2-methylphenyl)-2-(methoxymethyl)-1H-benzimidazole-4-carboxamide,
(102) 6-{[(2-bromo-6-fluorophenyl)carbonyl]amino}-N-(3-chloro-2-methylphenyl)-2-(methoxymethyl)-1H-benzimidazole-4-carboxamide,
(103) N-(3-chloro-2-methylphenyl)-6-{[(2-chloro-6-methylphenyl)carbonyl]amino}-2-(methoxymethyl)-1H-benzimidazole-4-carboxamide,
(104) N-(3-chloro-2-methylphenyl)-6-{[(2-chloro-4-methylphenyl)carbonyl]amino}-2-(methoxymethyl)-1H-benzimidazole-4-carboxamide,
(105) 6- {[(5-bromo-2-chlorophenyl)carbonyl]amino} -N-(3-chloro-2-methylphenyl)-2-(methoxymethyl)-1H-benzimidazole-4-carboxamide,
(106) 6-{[(2-bromo-5 -chlorophenyl)carbonyl]amino} -N-(3-chloro-2-methylphenyl)-2-(methoxymethyl)-1H-benzimidazole-4-carboxamide,
(107) N-(3-chloro-2-methylphenyl)-6-{[(2-chloro-5-methylphenyl)carbonyl]amino}-2-(methoxymethyl)-1H-benzimidazole-4-carboxamide,
(108) N-(3-chloro-2-methylphenyl)-2-(methoxymethyl)-6-({[5-methyl-2-(trifluoromethyl)phenyl]carbonyl}amino)-1H-benzimidazole-4-carboxamide,
(109) 6-({[2,5-bis(trifluoromethyl)phenyl]carbonyl}amino)-N-(3-chloro-2-methylphenyl)-2-(methoxymethyl)-1H-benzimidazole-4-carboxamide,
(110) 6-({[2,4-bis(trifluoromethyl)phenyl]carbonyl}amino)-N-(3-chloro-2-methylphenyl)-2-(methoxymethyl)-1H-benzimidazole-4-carboxamide,
(111) N-(3-chloro-2-methylphenyl)-6-({[5-fluoro-2-(trifluoromethyl)phenyl]carbonyl}amino)-2-(methoxymethyl)-1H-benzimidazole-4-carboxamide,
(112) N-(3-chloro-2-methylphenyl)-6-({[2-chloro-6-(trifluoromethyl)phenyl]carbonyl}amino)-2-(methoxymethyl)-1H-benzimidazole-4-carboxamide,
(113) N-(3-chloro-2-methylphenyl)-6-[({2-chloro-5-[2-(propan-2-yloxy)ethoxy]phenyl}carbonyl)amino]-2-(methoxymethyl)-1H-benzimidazole-4-carboxamide,
(114) 6-({[2-chloro-5-(2-ethoxyethoxy)phenyl] carbonyl} amino)-N-(3-chloro-2-methylphenyl)-2-(methoxymethyl)-1H-benzimidazole-4-carboxamide,
(115) 6-({[2-chloro-5-(3-methoxypropyl)phenyl]carbonyl}amino)-N-(3-chloro-2-methylphenyl)-2-(methoxymethyl)-1H-benzimidazole-4-carboxamide,
(116) 6-({[5-(3-tert-butoxyprop-1-yn-1-yl)-2-chlorophenyl]carbonyl}amino)-N-(3-chloro-2-methylphenyl)-2-(methoxymethyl)-1H-benzimidazole-4-carboxamide,

(117) 6-({[5-(3-tert-butoxypropyl)-2-chlorophenyl]carbonyl}amino)-N-(3-chloro-2-methylphenyl)-2-(methoxymethyl)-1H-benzimidazole-4-carboxamide,
(118) 6-({[2-chloro-5-(3-hydroxy-3-methylbutyl)phenyl]carbonyl}amino)-N-(3-chloro-2-methylphenyl)-2-(methoxymethyl)-1H-benzimidazole-4-carboxamide,
(119) 6-({[2-chloro-5-(ethoxymethyl)phenyl]carbonyl}amino)-N-(3-chloro-2-methylphenyl)-2-(methoxymethyl)-1H-benzimidazole-4-carboxamide,
(120) 6-[({2-chloro-5-[(2-ethoxyethoxy)methyl]phenyl}carbonyl)amino]-N-(3-chloro-2-methylphenyl)-2-(methoxymethyl)-1H-benzimidazole-4-carboxamide,
(121) 6-({[2-chloro-5-(2-cyclopropylethyl)phenyl]carbonyl}amino)-N-(3-chloro-2-methylphenyl)-2-(methoxymethyl)-1H-benzimidazole-4-carboxamide,
(122) N-(3-chloro-2-methylphenyl)-6-({[2-chloro-5-(2-phenylethyl)phenyl]carbonyl}amino)-2-(methoxymethyl)-1H-benzimidazole-4-carboxamide,
(123) N-(3-chloro-2-methylphenyl)-2-cyclopentyl-6-({[2-(trifluoromethyl)phenyl]carbonyl}amino)-1H-benzimidazole-4-carboxamide,
(124) N-(3-chloro-2-methylphenyl)-2-cyclopentyl-6-{ [(2,5-dichlorophenyl)carbonyl]amino}-1H-benzimidazole-4-carboxamide,
(125) 6- {[(2-chloro-6-fluorophenyl)carbonyl]amino} -N-(3-chloro-2-methylphenyl)-2-cyclopentyl-1H-benzimidazole-4-carboxamide,
(126) 6-{[(2-chloro-6-fluorophenyl)carbonyl]amino}-N-(3-chloro-2-methylphenyl)-2-[(2R)-tetrahydrofuran-2-yl]-1H-benzimidazole-4-carboxamide,
(127) N-(3-chloro-2-methylphenyl)-6- {[(2,6-dichlorophenyl)carbonyl]amino} -2-[(2R)-tetrahydrofuran-2-yl]-1H-benzimidazole-4-carboxamide,
(128) N-(3-chloro-2-methylphenyl)-6- {[(2,5-dichlorophenyl)carbonyl]amino} -2-[(2R)-tetrahydrofuran-2-yl]-1H-benzimidazole-4-carboxamide,
(129) N-(3-chloro-2-methylphenyl)-2-[(2S)-5-oxopyrrolidin-2-yl]-6-({[2-(trifluoromethyl)phenyl]carbonyl}amino)-1H-benzimidazole-4-carboxamide,
(130) N-(3 -chloro-2-methylphenyl)-2- [(2R)-5-oxopyrrolidin-2-yl]-6-({[2-(trifluoromethyl)phenyl]carbonyl}amino)-1H-benzimidazole-4-carboxamide,
(131) N-(3-chloro-2-methylphenyl)-2-[2-oxo-2-(pyrrolidin-1-yl)ethyl]-6-({[2-(trifluoromethyl)phenyl]carbonyl}amino)-1H-benzimidazole-4-carboxamide,
(132) N-(3-chloro-2-methylphenyl)-2-[2-(dimethylamino)-2-oxoethyl]-6-({[2-(trifluoromethyl)phenyl]carbonyl}amino)-1H-benzimidazole-4-carboxamide,
(133) N-(3-chloro-2-methylphenyl)-2-[2-(methylamino)-2-oxoethyl]-6-({[2-(trifluoromethyl)phenyl]carbonyl}amino)-1H-benzimidazole-4-carboxamide,
(134) 2-chloro-N-(3-chloro-2-methylphenyl)-6-({[2-(trifluoromethyl)phenyl]carbonyl}amino)-1H-benzimidazole-4-carboxamide,
(135) N-(3-chloro-2-methylphenyl)-2-[(2-methoxyethyl)amino]-6-({[2-(trifluoromethyl)phenyl]carbonyl}amino)-1H-benzimidazole-4-carboxamide,
(136) N-(3-chloro-2-methylphenyl)-2-[(2-hydroxyethyl)amino]-6-({[2-(trifluoromethyl)phenyl]carbonyl}amino)-1H-benzimidazole-4-carboxamide,
(137) N-(3-chloro-2-methylphenyl)-2-(methylamino)-6-({[2-(trifluoromethyl)phenyl]carbonyl}amino)-1H-benzimidazole-4-carboxamide,
(138) N-(3-chloro-2-methylphenyl)-2-(ethylamino)-6-({[2-(trifluoromethyl)phenyl]carbonyl}amino)-1H-benzimidazole-4-carboxamide,
(139) N-(3-chloro-2-methylphenyl)-2-[(2,2-dimethylpropyl)amino]-6-({[2-(trifluoromethyl)phenyl]carbonyl}amino)-1H-benzimidazole-4-carboxamide,
(140) N-(3-chloro-2-methylphenyl)-2-(cyclopentylamino)-6-({[2-(trifluoromethyl)phenyl]carbonyl}amino)-1H-benzimidazole-4-carboxamide,
(141) N-(3 -chloro-2-methylphenyl)-2-(piperidin-1-yl)-6-({[2-(trifluoromethyl)phenyl]carbonyl}amino)-1H-benzimidazole-4-carboxamide,
(142) N-(3-chloro-2-methylphenyl)-2-(4-methylpiperazin-1-yl)-6-({[2-(trifluoromethyl)phenyl]carbonyl}amino)-1H-benzimidazole-4-carboxamide,
(143) 2-[bis(2-hydroxyethyl)amino]-N-(3-chloro-2-methylphenyl)-6-({[2-(trifluoromethyl)phenyl]carbonyl} amino)-1H-benzimidazole-4-carboxamide,
(144) N-(3-chloro-2-methylphenyl)-2-(dimethylamino)-6-({[2-(trifluoromethyl)phenyl]carbonyl}amino)-1H-benzimidazole-4-carboxamide,
(145) N-(3-chloro-2-methylphenyl)-2- {[2-(morpholin-4-yl)ethyl]amino} -6-({[2-(trifluoromethyl)phenyl]carbonyl}amino)-1H-benzimidazole-4-carboxamide,

(146) N-(3-chloro-2-methylphenyl)-2-{[2-(dimethylamino)ethyl]amino} -6-({[2-(trifluoromethyl)phenyl]carbonyl}amino)-1H-benzimidazole-4-carboxamide,
(147) N-(3-chloro-2-methylphenyl)-2-(3-hydroxyazetidin-1-yl)-6-(1[2-(trifluoromethyl)phenyl]carbonyl}amino)-1H-benzimidazole-4-carboxamide,
(148) N-(3-chloro-2-methylphenyl)-2-[(3S)-3-(dimethylamino)pyrrolidin-1-yl]-6-({[2-(trifluoromethyl)phenyl]carbonyl}amino)-1H-benzimidazole-4-carboxamide,
(149) N-(3-chloro-2-methylphenyl)-2-[(3S)-3-hydroxypyrrolidin-1-yl] -6-({[2-(trifluoromethyl)phenyl]carbonyl}amino)-1H-benzimidazole-4-carboxamide,
(150) N-(3-chloro-2-methylphenyl)-2-{[2-(diethylamino)ethyl]amino}-6-({[2-(trifluoromethyl)phenyl]carbonyl}amino)-1H-benzimidazole-4-carboxamide,
(151) N-(3 -chloro-2-methylphenyl)-2-{[2-(pyrrolidin-1-yl)ethyl]amino}-6-({[2-(trifluoromethyl)phenyl]carbonyl]amino)-1H-benzimidazole-4-carboxamide,
(152) N-(3-chloro-2-methylphenyl)-2-{[3-(dimethylamino)propyl]amino}-6-({[2-(trifluoromethyl)phenyl]carbonyl}amino)-1H-benzimidazole-4-carboxamide,
(153) N-(3-chloro-2-methylphenyl)-2-{[3-(dimethylamino)-2,2-dimethylpropyl]amino}-6-({[2-(trifluoromethyl)phenyl]carbonyl}amino)-1H-benzimidazole-4-carboxamide,
(154) N-(3-chloro-2-methylphenyl)-2-{[2-(dipropan-2-ylamino)ethyl]amino}-6-({[2-(trifluoromethyl)phenyl]carbonyl}amino)-1H-benzimidazole-4-carboxamide,
(155) N-(3-chloro-2-methylphenyl)-2-(morpholin-4-yl)-6-({[2-(trifluoromethyl)phenyl]carbonyl}amino)-1H-benzimidazole-4-carboxamide,
(156) 2-amino-N-(3-chloro-2-methylphenyl)-6-({[2-(trifluoromethyl)phenyl]carbonyl}amino)-1H-benzimidazole-4-carboxamide,
(157) N-(3-chloro-2-methylphenyl)-2-[(3-hydroxy-2,2-dimethylpropyl)amino]-6-({[2-(trifluoromethyl)phenyl]carbonyl}amino)-1H-benzimidazole-4-carboxamide,
(158) N-(3-chloro-2-methylphenyl)-2-{[(3-methyloxetan-3-yl)methyl]amino}-6-({[2-(trifluoromethyl)phenyl]carbonyl}amino)-1H-benzimidazole-4-carboxamide,
(159) tert-butyl N-{4-[(3-chloro-2-methylphenyl)carbamoyl]-6-({[2-(trifluoromethyl)phenyl]carbonyl } amino)-1H-benzimidazol-2-yl}glycinate,
(160) N-{4-[(3-chloro-2-methylphenyl)carbamoyl]-6-({[2-(trifluoromethyl)phenyl]carbonyl}amino)-1H-benzimidazol-2-yl} glycine,
(161) N-(3-chloro-2-methylphenyl)-2-[(3-hydroxy-2,2-dimethylpropyl)amino]-1 - methyl-6-({[2-(trifluoromethyl)phenyl]carbonyl} amino)-1H-benzimidazole-4-carboxamide,
(162) N-(3-chloro-2-methylphenyl)-2-[(3-methoxy-2,2-dimethylpropyl)amino]-6-({[2-(trifluoromethyl)phenyl]carbonyl)amino)-1H-benzimidazole-4-carboxamide,
(163) N-(3-chloro-2-methylphenyl)-2-(pyrrolidin-1-yl)-6-({[2-(trifluoromethyl)phenyl]carbonyl}amino)-1H-benzimidazole-4-carboxamide,
(164) 2-(azetidin-1-yl)-N-(3-chloro-2-methylphenyl)-6-({[2-(trifluoromethyl)phenyl]carbonyl}amino)-1H-benzimidazole-4-carboxamide,
(165) N-(3-chloro-2-methylphenyl)-2-(3-methoxyazetidin-1-yl)-6-({[2-(trifluoromethyl)phenyl]carbonyl}amino)-1H-benzimidazole-4-carboxamide,
(166) N-(3-chloro-2-methylphenyl)-2-[(2-hydroxy-2-methylpropyl)amino]-6-({[2-(trifluoromethyl)phenyl]carbonyl}amino)-1H-benzimidazole-4-carboxamide,
(167) N-(3-chloro-2-methylphenyl)-2-{[(2 S)-tetrahydrofuran-2-ylmethyl]amino} -6-({ [2-(trifluoromethyl)phenyl]carbonyl}amino)-1H-benzimidazole-4-carboxamide,
(168) N-(3-chloro-2-methylphenyl)-2-{[(2R)-tetrahydrofuran-2-ylmethyl]amino}-6-({[2-(trifluoromethyl)phenyl]carbonyl}amino)-1H-benzimidazole-4-carboxamide,
(169) N-(3-chloro-2-methylphenyl)-2- {[(2S)-1-hydroxy-3-methylbutan-2-yl]amino}-6-({[2-(trifluoromethyl)phenyl]carbonyl}amino)-1H-benzimidazole-4-carboxamide,
(170) N-(3-chloro-2-methylphenyl)-2-{[(2R)-1-hydroxy-3-methylbutan-2-yl]amino}-6-({[2-(trifluoromethyl)phenyl]carbonyl}amino)-1H-benzimidazole-4-carboxamide,
(171) N-(3-chloro-2-methylphenyl)-2-{[(2S)-1-hydroxy-3,3-dimethylbutan-2-yl]amino}-6-({[2-(trifluoromethyl)phenyl]carbonyl}amino)-1H-benzimidazole-4-carboxamide,
(172) N-(3-chloro-2-methylphenyl)-2-[(3-methoxy-2,2-dimethylpropyl)(methyl)amino]-6-({[2-(trifluoromethyl)phenyl]carbonyl}amino)-1H-benzimidazole-4-carboxamide,
(173) N-(3-chloro-2-methylphenyl)-2-[(3-methoxypropyl)amino]-6-({[2-(trifluoromethyl)phenyl]carbonyl}amino)-1H-benzimidazole-4-carboxamide,
(174) N-(3-chloro-2-methylphenyl)-2-{[2-(propan-2-yloxy)ethyl]amino}-6-({[2-(trifluoromethyl)phenyl]carbonyl}amino)-1H-benzimidazole-4-carboxamide,

(175) 2-[(2-tert-butoxyethyl)amino]-N-(3-chloro-2-methylphenyl)-6-({[2-(trifluoromethyl)phenyl]carbonyl}amino)-1H-benzimidazole-4-carboxamide,

(176) N-(3-chloro-2-methylphenyl)-2-[(2-methoxy-2-methylpropyl)amino]-6-({[2-(trifluoromethyl)phenyl]carbonyl}amino)-1H-benzimidazole-4-carboxamide,

(177) N-(3-chloro-2-methylphenyl)-2- {[2-(methylsulfanyl)ethyl]amino} -6-({[2-(trifluoromethyl)phenyl]carbonyl}amino)-1H-benzimidazole-4-carboxamide,

(178) N-(3-chloro-2-methylphenyl)-2-(methylsulfanyl)-6-({[2-(trifluoromethyl)phenyl]carbonyl}amino)-1H-benzimidazole-4-carboxamide,

(179) N-(3-chloro-2-methylphenyl)-2-(methylsulfonyl)-6-({[2-(trifluoromethyl)phenyl]carbonyl}amino)-1H-benzimidazole-4-carboxamide,

(180) N-(3-chloro-2-methylphenyl)-2-(methylsulfinyl)-6-({[2-(trifluoromethyl)phenyl]carbonyl} amino)-1H-benzimidazole-4-carboxamide,

(181) 6- {[(2-chloro-6-fluorophenyl)carbonyl]amino} -N-(3-chloro-2-methylphenyl)-2-(dimethylamino)-1H-benzimidazole-4-carboxamide,

(182) N-(3-chloro-2-methylphenyl)-6-{[(2,6-dichlorophenyl)carbonyl]amino}-2-(dimethylamino)-1H-benzimidazole-4-carboxamide,

(183) N-(3-chloro-2-methylphenyl)-6-{[(2,4-dichlorophenyl)carbonyl]amino}-2-(dimethylamino)-1H-benzimidazole-4-carboxamide,

(184) N-(3-chloro-2-methylphenyl)-6-{[(2,5-dichlorophenyl)carbonyl]amino}-2-(dimethylamino)-1H-benzimidazole-4-carboxamide,

(185) 6-{[(2-bromo-6-fluorophenyl)carbonyl]amino}-N-(3-chloro-2-methylphenyl)-2-(dimethylamino)-1H-benzimidazole-4-carboxamide,

(186) 6-{[(2-bromo-6-chlorophenyl)carbonyl]amino}-N-(3-chloro-2-methylphenyl)-2-(dimethylamino)-1H-benzimidazole-4-carboxamide,

(187) 6-({[2-chloro-5-(cyclopropylethynyl)phenyl]carbonyl}amino)-N-(3-chloro-2-methylphenyl)-2-(dimethylamino)-1H-benzimidazole-4-carboxamide,

(188) N-(3-chloro-2-methylphenyl)-6-{[(2,5-dichlorophenyl)carbonyl]amino}-2-[(3-hydroxy-2,2-dimethylpropyl)amino]-1H-benzimidazole-4-carboxamide,

(189) N-(3 -chloro-2-methylphenyl)-6- {[(2,5-dichlorophenyl)carbonyl]amino} -2-[(3-methoxy-2,2-dimethylpropyl)amino]-1H-benzimidazole-4-carboxamide,

(190) N-(3-chloro-2-methylphenyl)-6-{[(2,5-dichlorophenyl)carbonyl]amino} -2-[(2-hydroxy-2-methylpropyl)amino]-1H-benzimidazole-4-carboxamide,

(191) N-(3-chloro-2-methylphenyl)-6-{[(2,5-dichlorophenyl)carbonyl]amino}-2-[(2-methoxy-2-methylpropyl)amino]-1H-benzimidazole-4-carboxamide,

(192) N-(3-chloro-2-methylphenyl)-6-{[(2,5-dichlorophenyl)carbonyl]amino}-2-{[2-(propan-2-yloxy)ethyl]amino}-1H-benzimidazole-4-carboxamide,

(193) 6-{[(2-chloro-6-fluorophenyl)carbonyl]amino}-N-(3-chloro-2-methylphenyl)-2-{[2-(propan-2-yloxy)ethyl]amino}-1H-benzimidazole-4-carboxamide,

(194) 2-[(2-tert-butoxyethyl)amino] -6- {[(2-chloro-6-fluorophenyl)carbonyl]amino}-N-(3-chloro-2-methylphenyl)-1H-benzimidazole-4-carboxamide,

(195) 6-{[(2-chloro-6-fluorophenyl)carbonyl]amino}-N-(3-chloro-2-methylphenyl)-2-[(3-methoxy-2,2-dimethylpropyl)amino]-1H-benzimidazole-4-carboxamide,

(196) 6-{[(2-chloro-6-fluorophenyl)carbonyl]amino}-N-(3-chloro-2-methylphenyl)-2-[(2-methoxy-2-methylpropyl)amino]-1H-benzimidazole-4-carboxamide,

(197) 6-{[(2-chloro-6-fluorophenyl)carbonyl]amino}-N-(3-chloro-2-methylphenyl)-2-{[(2S)-tetrahydrofuran-2-ylmethyl]amino}-1H-benzimidazole-4-carboxamide,

(198) 6-{[(2-chloro-6-fluorophenyl)carbonyl]amino}-N-(3-chloro-2-methylphenyl)-2-{[(2R)-tetrahydrofuran-2-ylmethyl]amino}-1H-benzimidazole-4-carboxamide,

(199) 6-{[(2-chloro-6-fluorophenyl)carbonyl]amino}-N-(3-chloro-2-methylphenyl)-2-[(3-hydroxy-2,2-dimethylpropyl)amino]-1H-benzimidazole-4-carboxamide,

(200) 6-{[(2-chloro-6-fluorophenyl)carbonyl]amino}-N-(3-chloro-2-methylphenyl)-2-{[(2S)-1-hydroxy-3-methylbutan-2-yl]amino}-1H-benzimidazole-4-carboxamide,

(201) N-(3-chloro-4-methylphenyl)-2-(dimethylamino)-6-({[2-(trifluoromethyl)phenyl]carbonyl} amino)-1H-benzimidazole-4-carboxamide,

(202) N-(4-tert-butylphenyl)-2-(dimethylamino)-6-({[2-(trifluoromethyl)phenyl]carbonyl}amino)-1H-benzimidazole-4-carboxamide,

(203) N-(2,3-dihydro-1H-inden-5-yl)-2-(dimethylamino)-6-({[2-(trifluoromethyl)phenyl]carbonyl}amino)-1H-benzimidazole-4-carboxamide,

(204) 6-{[(2-chloro-6-fluorophenyl)carbonyl]amino}-N-(3-chloro-4-methylphenyl)-2-(dimethylamino)-1H-benzimidazole-4-carboxamide,

(205) N-(3-chloro-4-methylphenyl)-6-{[(2,6-dichlorophenyl)carbonyl]amino}-2-(dimethylamino)-1H-benzimidazole-4-carboxamide,

(206) N-(3-chloro-4-methylphenyl)-6-{[(2,5-dichlorophenyl)carbonyl]amino}-2-(dimethylamino)-1H-benzimidazole-4-carboxamide,

(207) N-(3-chloro-2-methylphenyl)-2-cyclopropyl-6-{[(2,5-dichlorophenyl)carbonyl]amino}-1H-benzimidazole-4-carboxamide,

(208) N-(3-chloro-4-methylphenyl)-2-cyclopropyl-6-{[(2,5-dichlorophenyl)carbonyl]amino}-1H-benzimidazole-4-carboxamide,

(209) N-(3-chloro-2-methylphenyl)-6-{[(2,5-dichlorophenyl)carbonyl]amino}-2-(1-methylcyclopropyl)-1H-benzimidazole-4-carboxamide,

(210) N-(3 -chloro-4-methylphenyl)-6- {[(2,5-dichlorophenyl)carbonyl]amino}-2-(1-methylcyclopropyl)-1H-benzimidazole-4-carboxamide,

(211) N-(3-chloro-2-methylphenyl)-2-(methoxymethyl)-6-({[2-(methylsulfonyl)phenyl]carbonyl}amino)-1H-benzimidazole-4-carboxamide,

(212) N-(3-chloro-4-methylphenyl)-6-{[(2,5-dichlorophenyl)carbonyl]amino}-2-(2-methoxyethyl)-1H-benzimidazole-4-carboxamide,

(213) 2-(methoxymethyl)-N-phenyl-6-({[2-(trifluoromethyl)phenyl] carbonyl}amino)-1H-benzimidazole-4-carboxamide,

(214) 2-(methoxymethyl)-N-propyl-6-({[2-(trifluoromethyl)phenyl]carbonyl}amino)-1H-benzimidazole-4-carboxamide,

(215) 2-(methoxymethyl)-N-(pyridin-3-yl)-6-({[2-(trifluoromethyl)phenyl]carbonyl}amino)-1H-benzimidazole-4-carboxamide,

(216) N-benzyl-2-(methoxymethyl)-6-({[2-(trifluoromethyl)phenyl]carbonyl}amino)-1H-benzimidazole-4-carboxamide,

(217) N-(cyclohexylmethyl)-2-(methoxymethyl)-6-({[2-(trifluoromethyl)phenyl]carbonyl}amino)-1H-benzimidazole-4-carboxamide,

(218) 2-(methoxymethyl)-N-(naphthalen-1-yl)-6-({[2-(trifluoromethyl)phenyl]carbonyl}amino)-1H-benzimidazole-4-carboxamide,

(219) 2-(methoxymethyl)-N-(thiophen-3-yl)-6-({[2-(trifluoromethyl)phenyl]carbonyl}amino)-1H-benzimidazole-4-carboxamide,

(220) N-(2,1,3-benzothiadiazol-4-yl)-2-(methoxymethyl)-6-({[2-(trifluoromethyl)phenyl]carbonyl}amino)-1H-benzimidazole-4-carboxamide,

(221) N-(1,1-dioxido-1-benzothiophen-6-yl)-2-(methoxymethyl)-6-({[2-(trifluoromethyl)phenyl]carbonyl}amino)-1H-benzimidazole-4-carboxamide,

(222) 2-(methoxymethyl)-N-(thiophen-2-ylmethyl)-6-({[2-(trifluoromethyl)phenyl]carbonyl)amino)-1H-benzimidazole-4-carboxamide,

(223) N-(1H-indol-5-yl)-2-(methoxymethyl)-6-({[2-(trifluoromethyl)phenyl]carbonyl}amino)-1H-benzimidazole-4-carboxamide,

(224) N-(1,3-benzothiazol-2-yl)-2-(methoxymethyl)-6-({[2-(trifluoromethyl)phenyl]carbonyl}amino)-1H-benzimidazole-4-carboxamide,

(225) N-(2,2-dimethylpropyl)-2-(methoxymethyl)-6-({[2-(trifluoromethyl)phenyl]carbonyl}amino)-1H-benzimidazole-4-carboxamide,

(226) 2-(methoxymethyl)-N-(thiophen-2-yl)-6-({[2-(trifluoromethyl)phenyl]carbonyl}amino)-1H-benzimidazole-4-carboxamide,

(227) N-(5-chloro-1,3-benzoxazol-2-yl)-2-(methoxymethyl)-6-({[2-(trifluoromethyl)phenyl] carbonyl}amino)-1H-benzimidazole-4-carboxamide,

(228) N-(2-benzylphenyl)-2-(methoxymethyl)-6-({[2-(trifluoromethyl)phenyl]carbonyl}amino)-1H-benzimidazole-4-carboxamide,

(229) 2-(methoxymethyl)-N-(quinolin-8-yl)-6-({[2-(trifluoromethyl)phenyl]carbonyl}amino)-1H-benzimidazole-4-carboxamide,

(230) N-(cycloheptylmethyl)-2-(methoxymethyl)-6-({[2-(trifluoromethyl)phenyl]carbonyl}amino)-1H-benzimidazole-4-carboxamide,

(231) N-(1,3-benzoxazol-2-yl)-2-(methoxymethyl)-6-({[2-(trifluoromethyl)phenyl]carbonyl}amino)-1H-benzimidazole-4-carboxamide,

(232) N-(6-chloro-1,3-benzoxazol-2-yl)-2-(methoxymethyl)-6-({[2-(trifluoromethyl)phenyl]carbonyl}amino)-1H-benzimidazole-4-carboxamide,

(233) N-[3-chloro-2-(hydroxymethyl)phenyl]-2-(methoxymethyl)-6-({[2-(trifluoromethyl)phenyl]carbonyl}amino)-1H-benzimidazole-4-carboxamide,
(234) N-(3-chloro-2-methylphenyl)-6- {[(3-fluoropyridin-2-yl)carbonyl]amino} -2-(methoxymethyl)-1H-benzimidazole-4-carboxamide,
(235) N-(3-chloro-2-methylphenyl)-6-{[(3-chloropyridin-4-yl)carbonyl]amino}-2-(methoxymethyl)-1H-benzimidazole-4-carboxamide,
(236) N-(3-chloro-2-methylphenyl)-6- {[(3,5-dichloropyridin-4-yl)carbonyl]amino}-2-(methoxymethyl)-1H-benzimidazole-4-carboxamide,
(237) 6-{[(5-butoxy-2-chlorophenyl)carbonyl]amino}-N-(3-chloro-2-methylphenyl)-2-(methoxymethyl)-1H-benzimidazole-4-carboxamide,
(238) 6-({[2-chloro-5-(2,2-difluoroethoxy)phenyl]carbonyl}amino)-N-(3-chloro-2-methylphenyl)-2-(methoxymethyl)-1H-benzimidazole-4-carboxamide, and
(239) N-(3-chloro-2-methylphenyl)-6-({[2-chloro-5-(4,4,4-trifluorobutoxy)phenyl]carbonyl}amino)-2-(methoxymethyl)-1H-benzimidazole-4-carboxamide,

or a tautomer of the compound, or a pharmaceutically acceptable salt thereof.
(9) The prophylactic and/or therapeutic agent for cancer according to any one of (2) to (8),
wherein the mPGES-1 inhibitor is a compound selected from the group consisting of:

N-(3-chloro-2-methylphenyl)-2-(methoxymethyl)-6-({[2-(trifluoromethyl)phenyl]carbonyl}amino)-1H-benzimidazole-4-carboxamide,
N-(3-chloro-2-methylphenyl)-2-(methoxymethyl)-6-({[2-(trifluoromethyl)phenyl]carbonyl}amino)-1H-benzimidazole-4-carboxamide hydrochloride,
N-(3-chloro-2-methylphenyl)-2-(methoxymethyl)-6-({[2-(trifluoromethyl)phenyl]carbonyl}amino)-1H-benzimidazole-4-carboxamide methanesulfonate,
N-(3-chloro-2-methylphenyl)-2-(methoxymethyl)-6-({[2-(trifluoromethyl)phenyl]carbonyl}amino)-1H-benzimidazole-4-carboxamide 4-methylbenzenesulfonate,
N-(3-chloro-2-methylphenyl)-2-(methoxymethyl)-6-({[2-(trifluoromethyl)phenyl]carbonyl}amino)-1H-benzimidazole-4-carboxamide sulfate,
N-(3-chloro-2-methylphenyl)-2-(1-methylcyclopropyl)-6-({[2-(trifluoromethyl)phenyl]carbonyl}amino)-1H-benzimidazole-4-carboxamide,
N-(3-chloro-2-methylphenyl)-2-(1-methylcyclopropyl)-6-({[2-(trifluoromethyl)phenyl]carbonyl}amino)-1H-benzimidazole-4-carboxamide hydrochloride,
N-(3-chloro-2-methylphenyl)-2-(1-methylcyclopropyl)-6-({[2-(trifluoromethyl)phenyl]carbonyl}amino)-1H-benzimidazole-4-carboxamide methanesulfonate,
N-(3-chloro-2-methylphenyl)-2-(1-methylcyclopropyl)-6-({[2-(trifluoromethyl)phenyl]carbonyl}amino)-1H-benzimidazole-4-carboxamide 4-methylbenzenesulfonate,
N-(3-chloro-2-methylphenyl)-2-(1-methylcyclopropyl)-6-({[2-(trifluoromethyl)phenyl]carbonyl}amino)-1H-benzimidazole-4-carboxamide sulfate,
N-(3-chloro-4-methylphenyl)-6-{[(2,5-dichlorophenyl)carbonyl]amino-2-(dimethylamino)-1H-benzimidazole-4-carboxamide,
N-(3-chloro-4-methylphenyl)-6-{[(2,5-dichlorophenyl)carbonyl]amino-2-(dimethylamino)-1H-benzimidazole-4-carboxamide hydrochloride,
N-(3-chloro-4-methylphenyl)-6- { [(2,5-dichlorophenyl)carbonyl]amino } -2-(dimethylamino)-1H-benzimidazole-4-carboxamide methanesulfonate,
N-(3-chloro-4-methylphenyl)-6-{[(2,5-dichlorophenyl)carbonyl]amino}-2-(dimethylamino)-1H-benzimidazole-4-carboxamide 4-methylbenzenesulfonate, and
N-(3-chloro-4-methylphenyl)-6-{[(2,5-dichlorophenyl)carbonyl]amino}-2-(dimethylamino)-1H-benzimidazole-4-carboxamide sulfate,
or a tautomer of the compound, or a pharmaceutically acceptable salt thereof.

(10) The prophylactic and/or therapeutic agent for cancer according to any one of the above-described (1) to (9), wherein the immune checkpoint inhibitor has a mode of action selected from the group consisting of adenosine A2A receptor antagonist, adenosine A2B receptor antagonist, anti-5'-nucleotidase (anti-NT5E, anti-CD73), anti-CD134 (antibody OX40), anti-CD154 (anti-CD40L), anti-CD223 (anti-LAG-3: Lymphocyte Activation Gene 3 Protein), anti-CD27, anti-CD276 antigen (anti-B7-H3), anti-CD70, anti-CTLA4: Cytotoxic T-Lymphocyte Protein 4 (anti-CD152), anti-DLL1: delta-Like Protein 1, anti-ENTPD1 (anti-CD39), anti-ILDR2: Immunoglobulin-Like Domain-Containing Receptor 2 (anti-Clorf32), anti-PD-1, anti-PD-LI (anti-CD274), anti-PVRIG: Transmembrane protein PVRIG, anti-TGFβ2: Transforming Growth Factor beta-2, anti-TIM3: Hepatitis A Virus Cellular Receptor 2, anti-VISTA: V-Type Immunoglobulin Domain-Containing Suppressor of T-cell Activation, antibody VTCN1 (anti-B7-H4, anti-Ovr110), CD47/SIRPalpha interaction inhibitor (Tyrosine-Protein Phosphatase Non-receptor Type Substrate 1), tryptophan-2,3-dioxygenase: TDO inhibition, and tubulin polymerization inhibition.

(11) The prophylactic and/or therapeutic agent for cancer according to any one of the above-described (1) to (9), wherein the immune checkpoint inhibitor is at least one selected from the group consisting of a 5'-nucleotidase inhibitor, an adenosine A2A receptor antagonist, an adenosine A2B receptor antagonist, an anti-CD73 antibody, an anti-CTLA-4/OX40 bispecific antibody, a PEGylated Fab antibody fragment against the CD40 ligand, an anti-CD40L Fc-fusion protein, an anti-CD40 ligand-Tn3 fusion protein, an anti-CD40L antibody, an anti-LAG-3 antibody, an anti-CD27 antibody, an anti-B7-H3 antibody, an anti-CD70 antibody, an anti-CTLA-4 antibody, a CTLA-4 targeting probody, an anti-CTLA-4/LAG-3 bispecific antibody, an anti-PD-Ll/CTLA-4 bispecific antibody, an anti-PD-1 antibody having anti-DLL1 action, an anti-CD39 antibody, an anti-ILDR2 antibody, an anti-PD-1 antibody, a fusion protein of B7-DC and an antibody Fc portion, an anti-PD-1/anti-LAG-3 dual-affinity re-targeting (DART) protein, an anti-PD-1/CTLA-4 bispecific antibody, an anti-PD-1/CTLA-4 bispecific antibody, an anti-PD-1/ICOS bispecific antibody, an anti-PD-1/TIM-3 bispecific antibody, an anti-PD-1/PD-L1 bispecific antibody, an anti-PD-Ll antibody, a protease-activated anti-PD-L1 antibody prodrug, an anti-PD-L1/CD137 bispecific antibody, an anti-LAG-3/PD-Ll bispecific antibody, an anti-PD-L1/TIM3 bispecific antibody, DuoBody-PD-L1x4-1BB (GEN-1046), an anti-PVRIG antibody, a bifunctional anti-PD-L1/TGFβ2 Trap fusion protein, an anti-TIM-3 antibody, a fully human Fc-engineered IgG1K antibody targeting co-inhibitory receptor T-cell immunoglobulin and mucin, an anti-VISTA antibody, an anti-B7-H4 antibody, an anti-SIRPa antibody, a drug-bound antibody, an agonist redirected checkpoint (ARC) fusion protein consisting of the extracellular domains of human programmed cell death 1 (PD-1; PDCD1; CD279) and tumor necrosis factor ligand superfamily member 4 (TNFSF4; OX40 ligand; OX40L; CD252), linked by a central Fc domain (PD1-Fc-OX40L), an IDO-1 inhibitor a PD-L1 inhibitor, a PD-L1/PD-L2/VISTA antagonist, and a JAK2/STAT3 inhibitor.

(12) The prophylactic and/or therapeutic agent for cancer according to any one of the above-described (1) to (9), wherein the cancer is leukemia, malignant lymphoma, multiple myeloma, myelodysplastic syndrome, head and neck cancer, esophageal cancer, esophageal adenocarcinoma, gastric cancer, duodenal cancer, colorectal cancer, colon cancer, rectal cancer, liver cancer, gall bladder/bile duct cancer, biliary tract cancer, pancreatic cancer, thyroid cancer, lung cancer, breast cancer, ovarian cancer, cervical cancer, uterine body cancer, endometrial cancer, vaginal cancer, vulvar cancer, renal cancer, renal pelvis/urinary tract cancer, renal pelvic/ureteral cancer, urothelial cancer, penal cancer, prostate cancer, testicular tumor, bone/soft tissue sarcoma, malignant bone tumor, skin cancer, thymoma, mesothelioma, or cancer of unknown primary.

(13) A prophylactic and/or therapeutic agent for cancer, containing an mPGES-1 inhibitor and an immune checkpoint inhibitor as active ingredients.

(14) A pharmaceutical composition for prevention and/or treatment of cancer, the pharmaceutical composition containing an mPGES-1 inhibitor and a pharmaceutically acceptable carrier and for use in combination with an immune checkpoint inhibitor.

(15) A pharmaceutical composition for prevention and/or treatment of cancer, the pharmaceutical composition containing an mPGES-1 inhibitor, an immune checkpoint inhibitor, and a pharmaceutically acceptable carrier.

(16) An mPGES-1 inhibitor for use in combination with an immune checkpoint inhibitor for prevention and/or treatment of cancer.

(17) Use of an mPGES-1 inhibitor for the manufacture of a medicine for use in combination with an immune checkpoint inhibitor in prevention and/or treatment of cancer.

(18) A method for preventing and/or treating cancer, the method comprising administering an mPGES-1 inhibitor and an immune checkpoint inhibitor in combination to a subject in need of the combination.

Each of the above-described elements can be arbitrarily selected and combined.

ADVANTAGEOUS EFFECTS OF INVENTION

According to the invention, a prophylactic and/or therapeutic agent for cancer containing an mPGES-1 inhibitor as an active ingredient, the agent for use in combination with an immune checkpoint inhibitor, and a prophylactic and/or therapeutic agent for cancer containing an mPGES-1 inhibitor and an immune checkpoint inhibitor as active ingredients, can be provided.

BRIEF DESCRIPTION OF DRAWINGS

Fig. 1 shows an effect of using compound 1 and anti-mouse PD-1 antibody in combination in an allograft model of mouse colorectal cancer cell line CT26. In the drawing, the combined use represents a group for combined use of compound 1 and anti-mouse PD-1 antibody.

Fig. 2 shows an effect of using compound 2 and anti-mouse PD-1 antibody in combination in an allograft model of mouse colorectal cancer cell line CT26. In the drawing, the combined use represents a group for combined use of compound 2 and anti-mouse PD-1 antibody.

Fig. 3 shows an effect of using compound 1, compound 2, and anti-mouse CTLA-4 antibody in combination in an allograft model of mouse colorectal cancer cell line CT26. In the drawing, the combined use represents a group for combined use of compound 1 and anti-mouse CTLA-4 antibody, and a group for combined use of compound 2 and anti-mouse CTLA-4 antibody.

DESCRIPTION OF EMBODIMENTS

Embodiments of the present invention is described.

According to an aspect of the invention, there is provided a prophylactic and/or therapeutic agent for chronic prostate/pelvic pain syndrome, the agent containing an mPGES-1 inhibitor as an active ingredient.

According to an aspect of the invention, regarding the mPGES-1 inhibitor, a commercially available compound and/or a compound that can be produced by a conventional method in the field of synthetic organic chemistry can be used.

According to an aspect of the invention, the mPGES-1 inhibitor can be used as it is as a medicine but can also be used in the form of a pharmaceutically acceptable salt thereof through a known method. Examples of such a salt include salts of mineral acids such as hydrochloric acid, hydrobromic acid, sulfuric acid, and phosphoric acid; and salts of organic acids such as acetic acid, citric acid, tartaric acid, maleic acid, succinic acid, fumaric acid, p-toluenesulfonic acid, benzenesulfonic acid, and methanesulfonic acid.

For example, hydrochloride of an mPGES-1 inhibitor can be obtained by dissolving the mPGES-1 inhibitor in an alcohol solution, an ethyl acetate solution, or a diethyl ether solution of hydrogen chloride.

According to an aspect of the invention, the mPGES-1 inhibitor may have asymmetric carbon, and each of optical isomers and mixtures thereof can all be used as the prophylactic and/or therapeutic agent of the invention. An optical isomer can be produced by, for example, optically resolving a racemate obtained in the same manner as in the Examples that are described below, by utilizing the basicity of the racemate and by using an optically active acid (tartaric acid, dibenzoyltartaric acid, mandelic acid, 10-camphor-sulfonic acid, or the like), or can be produced by using an optically active compound that has been prepared in advance as a raw material. In addition to that, an optical isomer can also be produced by optical resolution using a chiral column or by asymmetric synthesis.

Furthermore, among mPGES-1 inhibitors, for those capable of forming tautomers, each of tautomers and mixtures thereof can all be used as the prophylactic and/or therapeutic agent of the invention.

According to an aspect of the invention, the mPGES-1 inhibitor is, for example, the above-described compound of Formula [I] (present compound) or a pharmaceutically acceptable salt thereof. The present compound can be produced from a known compound and/or an intermediate that can be easily synthesized, according to the method described in WO 2013/024898 and/or a known method.

With regard to the compound of Formula [I] (present compound), examples of each substituent are as follows.

Examples of the "halogen" include fluorine, chlorine, bromine, and iodine.

Examples of the "alkyl" include linear or branched alkyl having I to 8 carbon atoms, and specific examples thereof include methyl, ethyl, n-propyl, isopropyl, n-butyl, isobutyl, sec-butyl, tert-butyl, n-pentyl, isopentyl, n-hexyl, isohexyl, n-heptyl, isoheptyl, and n-octyl. Among them, alkyl having 1 to 6 carbon atoms is preferred, and alkyl having 1 to 3 carbon atoms is more preferred.

Examples of the alkyl moiety of "monoalkylamino", "dialkylamino", "monoalkylaminocarbonyl", "dialkylaminocarbonyl", "alkylcarbonyloxy", "alkyloxycarbonyl", "alkylcarbonyl", "alkylthio", "alkylsulfonyl", "alkylsulfinyl", "alkoxyalkyl", and "alkoxyalkylamino" include those similar to the above-described "alkyl".

Examples of the alkoxy moiety of "alkoxy" include linear or branched alkoxy having 1 to 8 carbon atoms, and specific examples thereof include methoxy, ethoxy, n-propoxy, isopropoxy, n-butoxy, isobutoxy, sec-butoxy, t-butoxy, n-pentyloxy, n-hexyloxy, n-heptyloxy, and n-octyloxy.

Examples of the alkoxy moiety of "alkoxyalkoxy", "alkoxyalkyl", and "alkoxyalkylamino" include those similar to the above-described "alkoxy".

The "heteroaryl" may be a monocyclic or bicyclic aromatic ring having one to three heteroatoms selected from the group consisting of a nitrogen atom, an oxygen atom, and a sulfur atom as constituent atoms. Specific examples thereof include furyl (for example, 2-furyl or 3-furyl), thienyl (for example, 2-thienyl or 3-thienyl), pyrrolyl (for example, 1-pyrrolyl, 2-pyrrolyl, or 3-pyrrolyl), imidazolyl (for example, 1-imidazolyl, 2-imidazolyl, or 4-imidazolyl), pyrazolyl (for example, 1-pyrazolyl, 3-pyrazolyl, or 4-pyrazolyl), triazolyl (for example, 1,2,4-triazol-1-yl, 1,2,4-triazol-3-yl, or 1,2,4-triazol-4-yl), tetrazolyl (for example, 1-tetrazolyl, 2-tetrazolyl, or 5-tetrazolyl), oxazolyl (for example, 2-oxazolyl, 4-oxazolyl,

or 5-oxazolyl), isoxazolyl (for example, 3-isoxazolyl, 4-isoxazolyl, or 5-isoxazolyl), oxadiazolyl (for example, 1,3,4-oxadiazol-2-yl), thiazolyl (for example, 2-thiazolyl, 4-thiazolyl, or 5-thiazolyl), thiadiazolyl (for example, 1,3,4-thiadiazolyl, 1,2,4-thiadiazolyl, or 1,2,3-thiadiazolyl), isothiazolyl (for example, 3-isothiazolyl, 4-isothiazolyl, or 5-isothiazolyl), pyridyl (for example, 2-pyridyl, 3-pyridyl, or o4-pyridyl), pyridazinyl (for example, 3-pyridazinyl or 4-pyridazinyl), pyrimidinyl (for example, 2-pyrimidinyl, 4-pyrimidinyl, or 5-pyrimidinyl), pyrazinyl (for example, 2-pyrazinyl), benzothiadiazolyl (for example, 1,2,3-benzothiadiazol-4-yl, 1,2,3-benzothiadiazol-5-yl, 2,1,3-benzothiadiazol-4-yl, or 2,1,3-benzothiadiazol-5-yl), benzothiazolyl (for example, benzothiazol-2-yl, benzothiazol-4-yl, benzothiazol-5-yl, benzothiazol-6-yl, or benzothiazol-7-yl), indolyl (for example, indol-3-yl, indol-4-yl, indol-5-yl, indol-6-yl, or indol-7-yl), benzothiophenyl (for example, 1-benzothiophen-2-yl, 1-benzothiophen-3-yl, 1-benzothiophen-4-yl, 1-benzothiophen-5-yl, 1-benzothiophen-6-yl, or 1-benzothiophen-7-yl), 1,1-dioxo-1-benzothiophenyl (for example, 1,1-dioxo-1-benzothiophen-2-yl, 1,1-dioxo-1-benzothiophen-3-yl, 1,1-dioxo-1-benzothiophen-4-yl, 1,1-dioxo-1-benzothiophen-5-yl, 1,1-dioxo-1-benzothiophen-6-yl, or 1,1-dioxo-1-benzothiophen-7-yl), quinolyl (quinolin-2-yl, quinolin-3-yl, quinolin-4-yl, quinolin-5-yl, quinolin-6-yl, quinolin-7-yl, or quinolin-8-yl), and 1,3-benzoxazol-2-yl.

Examples of the heteroaryl moiety of "heteroarylmethyl" include those similar to the above-described "heteroaryl".

Examples of the "saturated cyclic amino" include a 4-membered to 7-membered saturated cyclic amino group having one or two N atoms, the cyclic amino group optionally having one O or S as a ring-constituting atom and optionally being substituted with oxo, and specific examples thereof include 1-azetidinyl, 1-pyrrolidinyl, 1-imiazolidinyl, piperidino, 1-piperazinyl, 1-tetrahydropyrimidinyl, 4-morpholino, 4-thiomorpholino, 1-homopiperazinyl, and 2-oxo-oxazolidin-3-yl.

Examples of the saturated cyclic amino moiety of "saturated cyclic aminocarbonyl" include those similar to the above-described "saturated cyclic amino".

Examples of the "saturated heterocyclic group" include a 4-membered to 6-membered saturated heterocyclic group having one N or O as a ring-constituting atom, and specific examples thereof include 2-pyrrolidinyl, 3-pyrrolidinyl, 2-piperidinyl, 3-piperidinyl, 4-piperidinyl, 2-oxetanyl, 3-oxetanyl, 2-tetrahydrofuranyl, and 3-tetrahydrofuranyl.

Examples of the "cycloalkyl" include cycloalkyl having 3 to 8 carbon atoms, and specific examples thereof include cyclopropyl, cyclobutyl, cyclopentyl, cyclohexyl, cycloheptyl, and cyclooctyl.

Examples of the cycloalkyl moiety of "cycloalkylmethyl" include those similar to the above-described "cycloalkyl".

Examples of "naphthyl" include 1-naphthyl and 2-naphthyl.

Examples of "pyridyl" include 2-pyridyl, 3-pyridyl, and 4-pyridyl.

Examples of "alkynyl" include linear or branched alkynyl having 2 to 6 carbon atoms. Specific examples include ethynyl, 1-propynyl, 1-butynyl, 1-pentynyl, 2-propynyl, 2-butynyl, 3-butynyl, 1-methyl-2-propynyl, 2-pentynyl, 3-pentynyl, and 4-pentynyl.

According to an aspect of the invention, the mPGES-1 inhibitor is, for example, each of the above-described compounds (1) to (239) or a tautomer of the compound, or a pharmaceutically acceptable salt thereof, and the mPGES-1 inhibitor is preferably

N-(3-chloro-2-methylphenyl)-2-(methoxymethyl)-6-({[2-(trifluoromethyl)phenyl]carbonyl}amino)-1H-benzimidazole-4-carboxamide,
N-(3-chloro-2-methylphenyl)-2-(1-methylcyclopropyl)-6-({[2-(trifluoromethyl)phenyl]carbonyl}amino)-1H-benzimidazole-4-carboxamide, or
N-(3-chloro-4-methylphenyl)-6-{[(2,5-dichlorophenyl)carbonyl]amino}-2-(dimethylamino)-1H-benzimidazole-4-carboxamide,
or a pharmaceutically acceptable salt thereof, and more preferably
N-(3-chloro-2-methylphenyl)-2-(methoxymethyl)-6-({[2-(trifluoromethyl)phenyl]carbonyl}amino)-1H-benzimidazole-4-carboxamide,
N-(3-chloro-2-methylphenyl)-2-(methoxymethyl)-6-({[2-(trifluoromethyl)phenyl]carbonyl}amino)-1H-benzimidazole-4-carboxamide hydrochloride,
N-(3-chloro-2-methylphenyl)-2-(methoxymethyl)-6-({[2-(trifluoromethyl)phenyl]carbonyl}amino)-1H-benzimidazole-4-carboxamide 4-methylbenzenesulfonate,
N-(3-chloro-2-methylphenyl)-2-(1-methylcyclopropyl)-6-({[2-(trifluoromethyl)phenyl]carbonyl}amino)-1H-benzimidazole-4-carboxamide hydrochloride, or
N-(3-chloro-4-methylphenyl)-6-{[(2,5-dichlorophenyl)carbonyl]amino}-2-(dimethylamino)-1H-benzimidazole-4-carboxamide hydrochloride.

When the present compound or a pharmaceutically acceptable salt thereof is administered as a medicine, the present compound or a pharmaceutically acceptable salt thereof is administered as a pharmaceutical composition containing the present compound or a pharmaceutically acceptable salt thereof as it is or in a pharmaceutically acceptable nontoxic and inert carrier, for example, at a proportion of 0.001% to 99.5%, and preferably 0.1% to 90%, to a mammal

including a human.

As the carrier, one or more kinds of diluents, fillers, and other auxiliary agents for formulation, which are solid, semi-solid, or liquid, are used. It is desirable that the pharmaceutical composition according to the invention is administered in a unit dosage form. The pharmaceutical composition can be administered by interstitial administration, peroral administration, intravenous administration, topical administration (percutaneous administration, ocular instillation, intraperitoneal, intraperitoneal, intrapleural, and the like), or transrectally. Further, the pharmaceutical composition is to be administered in a dosage form appropriate for these administration methods.

It is desirable to adjust the dosage as a medicine after taking the conditions of the patient, such as age, body weight, the type and severity of the disease, the route of administration, the type of the compound of the invention, whether it is a salt or not, the type of the salt, and the like into consideration; however, usually, as the amount as an active ingredient of the compound of the invention or a pharmaceutically acceptable salt thereof for an adult, in the case of oral administration, the dosage is appropriately in the range of 0.01 mg to 5 g/adult, and preferably in the range of 1 mg to 1000 mg/adult, per day. In some cases, a dosage equal to or less than this may be sufficient, or in contrast, a dosage greater than this may be needed. Usually, the compound or a pharmaceutically acceptable salt thereof can be administered once or in several divided doses a day, or in the case of intravenous administration, the compound or a pharmaceutically acceptable salt thereof can be rapidly administered or can be administered continuously within 24 hours.

According to an aspect of the invention, the immune checkpoint inhibitory drug is not particularly limited as long as it is a substance capable of suppressing the function (signal) of an immune checkpoint molecule and an inhibitory drug for an immune checkpoint molecule.

According to an aspect of the invention, the immune checkpoint molecule means a molecule that exhibits an immunosuppressive function by transmitting co-inhibitory signals.

According to an aspect of the invention, examples of the immune checkpoint molecule include an adenosine A2A receptor, an adenosine A2B receptor, 5'-nucleotidase (NT5E, CD73), CD134 (OX40), CD154 (CD40L), CD223 (LAG-3: Lymphocyte Activation Gene 3 Protein), anti-CD27, anti-CD276 antigen (anti-B7-H3), anti-CD70, CTLA-4: Cytotoxic T-Lymphocyte Protein 4 (anti-CD152), DLL1: delta-Like Protein 1, ENTPD1 (CD39), ILDR2: Immunoglobulin-Like Domain-Containing Receptor 2 (C1orf32), PD-1, PD-L1 (CD274), PVRIG: Transmembrane protein PVRIG, TGFβ2: Transforming Growth Factor beta-2, TIM3: Hepatitis A Virus Cellular Receptor 2, VISTA: V-Type Immunoglobulin Domain-Containing Suppressor of T-cell Activation, VTCN1 (anti-B7-H4, Ovr110), CD47/SIRPalpha (Tyrosine-Protein Phosphatase Non-receptor Type Substrate 1), tryptophan-2,3-dioxygenase: TDO, and tubulin, and preferred examples include CTLA-4, PD-1, PD-L1 (programmed cell death-ligand 1), PD-L2 (programmed cell death-ligand 2), LAG-3 (Lymphocyte activation gene 3), TIM3 (T cell immunoglobulin and mucin-3), BTLA (B and T lymphocyte attenuator), B7H3, B7H4, CD160, CD39, CD73, A2aR (adenosine A2a receptor), KIR (killer inhibitory receptor), VISTA (V-domain Ig-containing suppressor of T cell activation), IDO1 (Indoleamine 2,3-dioxygenase), Arginase I, TIGIT (T cell immunoglobulin and ITIM domain), and CD115; however, the immune checkpoint molecule is not particularly limited as long as it is equivalent to a molecule that exhibits an immunosuppressive function by transmitting a co-inhibitory signal.

According to an aspect of the invention, examples of the immune checkpoint inhibitory drug include immune checkpoint inhibitory drugs having a mode of action selected from the group consisting of adenosine A2A receptor antagonist, adenosine A2B receptor antagonist, anti-5'-nucleotidase (anti-NT5E, anti-CD73), anti-CD 134 (anti-OX40), anti-CD154 (anti-CD40L), anti-CD223 (anti-LAG-3), anti-CD27, anti-CD276 antigen (anti-B7-H3), anti-CD70, anti-CTLA4 (anti-CD152), anti-DLL1, anti-ENTPD1 (anti-CD39), anti-ILDR2 (anti-C1orf32), anti-PD-1, anti-PD-L1 (anti-CD274), anti-PVRIG, anti-TGFβ2, anti-TIM3, anti-VISTA, antibody VTCN1 (anti-B7-H4, anti-Ovr110), CD47/SIRPalpha interaction inhibition, tryptophan-2,3-dioxygenase: TDO inhibition, and tubulin polymerization inhibition, and preferred examples include immune checkpoint inhibitory drugs having a mode of action selected from the group consisting of anti-CTLA-4, anti-PD-1, anti-PD-L1, anti-PD-L2, anti-LAG-3, anti-TIM3, anti-BTLA, anti-B7H3, anti-B7H4, anti-CD160, anti-CD39, anti-CD73, anti-A2aR, anti-KIR, anti-VISTA, anti-IDO1, anti-Arginase I, anti-TIGIT, and anti-CD 115.

According to an aspect of the invention, the immune checkpoint inhibitory drug is, for example, an inhibitory drug for a human immune checkpoint molecule, and preferably, a neutralizing antibody to the human immune checkpoint molecule may be mentioned.

According to an aspect of the invention, examples of the neutralizing antibody to the human immune checkpoint molecule include mab (whole monoclonal antibody), Fab (antigen binding region fragment, one arm), $F(ab')_2$ (antigen binding region fragment including a hinge region, both arms), Fab' (antigen binding region fragment including a hinge region, one arm), scFv (single-stranded variable region fragment), di-scFv (dimer single-stranded variable region fragment), sdAb (single-domain antibody, nanoantibody), and bispecific monoclonal antibody.

According to an aspect of the invention, the neutralizing antibody to the human immune checkpoint molecule is a bispecific monoclonal antibody and is an artificial protein that is composed of fragments of two different monoclonal antibodies and binds to two different types of antigens, and examples thereof include F(ab')2, BilE, IgG-IgG, Bi-sdAb, CrossMab, TandAb, DART, DVD-Ig, TrioMab, and Triplebody.

Examples of the immune checkpoint inhibitory drug are mentioned below; however, the immune checkpoint

inhibitory drug is not limited to these.

As an aspect of the invention, examples of the immune checkpoint inhibitor, which is a 5'-nucleotidase (CD73) inhibitor, include MCI-186; MT-1186; TW-001; 5798V6YJRP (UNII code); SIM-071201; Y-2; and AB-680.

As an aspect of the invention, examples of the immune checkpoint inhibitor, which is an adenosine A2A receptor antagonist, include CPI-444; V-81444; 8KFO2187CP (UNII code); NIR-178; PBF-509; AZD-4635; and HTL-1071.

As an aspect of the invention, examples of the immune checkpoint inhibitor, which is as an adenosine A2B receptor antagonist, include AB-928.

As an aspect of the invention, examples of the immune checkpoint inhibitor having anti-5'-nucleotidase (NT5E, CD73) action include MEDI-9447; 5CRY01URYQ (UNII code); BMS-986179; CPI-006; CPX-006; NZV-930; SRF-373; TJ-004309; TJ-4309; and TJD-5.

As an aspect of the invention, examples of the immune checkpoint inhibitor having anti-CD154 (anti-CD40L) action include CDP-7657; BMS-986004; 449MIE2SD6 (UNII code); MEDI-4920; VIB-4920; INX-021; SAR-441344; and AT-1501.

As an aspect of the invention, examples of the immune checkpoint inhibitor having anti-CD223 (anti-LAG-3) action include IMP-701; ImmuTune IMP-701; LAG-525; BMS-986016; ONO-4482; AF75XOF6W3 (UNII code); 2831781; GSK-2831781; TSR-033; MK-4280; BI-754111; REGN-3767; OX5LRQ5H6K (UNII code); Sym-022; INCAGN-02385; and INCAGN-2385.

As an aspect of the invention, examples of the immune checkpoint inhibitor having anti-CD27 action include CDX-1127.

As an aspect of the invention, examples of the immune checkpoint inhibitor having anti-CD276 antigen (B7-H3) action include 8H9; MAb8H9; 131I-8H9; MGA-271; M6030H73N9 (UNII code); 124I-8H9; and MGD-009.

As an aspect of the invention, examples of the immune checkpoint inhibitor having anti-CD70 action include ARGX-110 and JNJ-4550.

As an aspect of the invention, examples of the immune checkpoint inhibitor having antibody CTLA-4 (anti-CD152) action include 0D1; BMS-734016; MAb10D14; MDX-010; MDX-CTLA4; MDX-101 (formerly); 4.1.1; CP-642570; CP-675206; CT-4.1.1; MEDI-1123; PF-06753388; AGEN-1884; BMS-986218; ADU-1604; BMS-986249; CS-1002; XmAb-22841; BCD-145; REGN-4659; KN-046; IBI-310; andAGEN-1181.

As an aspect of the invention, examples of the immune checkpoint inhibitor having anti-DLL1 action include CT-011 and MDV-9300.

As an aspect of the invention, examples of the immune checkpoint inhibitor having anti-ENTPD1 (anti-CD39) action include TTX-030.

As an aspect of the invention, examples of the immune checkpoint inhibitor having anti- ILDR2 (anti-Clorf32) action include BAY-1905254.

As an aspect of the invention, examples of the immune checkpoint inhibitor having anti-PD-1 action include BMS-936558; MDX-1106; ONO-4538; MK-3475; SCH-900475; h409A11; 2661380; AMP-224; B7-DCIg; ANB-011; TSR-042; WBP-285; P0GVQ9A4S5 (UNII code); BGB-A317; hu317-1/IgG4mt2; 0KVO411B3N (UNII code); REGN-2810; SAR-439684; 6QVL057INT (UNII code); PDR-001; HR-301210; INCSHR-01210; SHR-1210; MGD-013; PF-06801591; RN-888; LZZ0IC2EWP (UNII code); BCD-100; XmAb-20717; JS-001; TAB-001; APL-501; CBT-501; GB-226; IBI-308; JNJ-3283; JNJ-63723283; LYK98WP91F (UNII code); BI-754091; CC-90006; NCMGA-00012; INCMGA-0012; MGA-012; ABBV-181; AGEN-2034w; GLS-010; WBP-3055; AK-104; LZM-009; Sym-021; AB-122; Pd-1-pik; AK-105; CS-1003; mAb-23104; JTX-4014; HLX-10; MEDI-5752; F-520; BMS-986213; RG-7769; RO-7121661; HX-008; IBI-318; BAT-1306; AMG-404; LY-3434172; 609-A; and ONO-4685.

As an aspect of the invention, examples of the immune checkpoint inhibitor having anti-PD-LI (anti-CD274) action include MPDL-3280A; RG-7446; RO-5541267; 52CMI0WC3Y (UNII code); 28X28X9OKV (UNII code); MEDI-4736; 451238; MSB-0010682; MSB-0010718C; PF-06834635; KXG2PJ551I (UNII code); STI-1014; STI-A1014; ZKAB-001; CK-301; TG-1501; CX-072; MCLA-145; LY-3300054; NR4MAD6PPB (UNII code); 3D-025; ASC-22; KN-035; APL-502; CBT-502; TQB-2450; CS-1001; WBP-3155; FAZ-053; FS-118; FS118mAb2; LAG-3/PD-L1mAb2; HTI-1088; SHR-1316; MSB-2311; IMC-001; STI-3031; STI-A-1015; STI-A1015; HLX-20; PL2#3; A-167; KL-A167; LY-3415244; Duo-Body-PD-L1x4-1BB; and GEN-1046.

As an aspect of the invention, examples of the immune checkpoint inhibitor having anti-PVRIG action include COM-701.

As an aspect of the invention, examples of the immune checkpoint inhibitor, which is anti-TGFbeta2, include M-7824 and MSB-0011359C.

As an aspect of the invention, examples of the immune checkpoint inhibitor having anti-TIM3 action include MBG-453; APE-5137; TSR-022; WBP-296A; 3K5H4TX2KP (UNII code); LY-3321367; BGB-A425; BMS-986258; ONO-7807; Sym-023; INCAGN-02390; and INCAGN-2390.

As an aspect of the invention, examples of the immune checkpoint inhibitor having anti-VISTA (anti-VSIR) action include JNJ-61610588 and 1UI8F5IIZ4 (UNII code).

As an aspect of the invention, examples of the immune checkpoint inhibitor having anti-VTCN1 (anti-B7-H4; anti-Ovr110) action include FPA-150.

As an aspect of the invention, examples of the immune checkpoint inhibitor, which is a CD47/SIRPalpha interaction inhibitor, include BI-765063; Effi-DEM; and OSE-172.

As an aspect of the invention, examples of the immune checkpoint inhibitor, which is a cytotoxic T cell protein 4 inhibitor, include MK-1308.

As an aspect of the invention, examples of the immune checkpoint inhibitor, which is a DNA alkylating drug, include MGC-018.

As an aspect of the invention, examples of the immune checkpoint inhibitor that acts on Tumor Necrosis Factor Receptor Superfamily Member 4 (OX40, CD134) include PD-1-Fc-OX40L; SL-279252; and TAK-252.

As an aspect of the invention, examples of the immune checkpoint inhibitor that targets Programmed Cell Death 1 Ligand 1 (PD-L1; CD274) include ES-101; INBRX-105; INBRX-105-1; and GS-4224.

As an aspect of the invention, examples of the immune checkpoint inhibitor, which is an indoleamine 2,3-dioxygenase 1 (IDO1; IDO) inhibitor, include D-1MT; NLG-8189; NSC-721782; INCB-024360; INCB-24360; 71596A9R13 (UNII code); BMS-986205; F-001287; ONO-7701; 0A7729F42K (as hydrochloride); EOS-200271; PF-06840003; KHK-2455; NLG-802; and LY-3381916.

As an aspect of the invention, examples of the immune checkpoint inhibitor, which is a Programmed Cell Death 1 (PDCD1; PD-1; CD279)/PD-1 Ligand 1 (PD-L1; CD274) interaction inhibitor, include INCB-086550.

As an aspect of the invention, examples of the immune checkpoint inhibitor, which is a Programmed Cell Death 1 Ligand 1 (PD-L1; CD274) antagonist, include AUPM-170 and CA-170.

As an aspect of the invention, examples of the immune checkpoint inhibitor, which is a Signal Transducer and Activator of Transcription 3 (STAT3) inhibitor, include WP-1066.

As an aspect of the invention, examples of the immune checkpoint inhibitor, which is a tryptophan-2,3-dioxygenase (TDO) inhibitor, include HTI-1090; SHR-9146; M-4112; DN-1406131; LPM-3480226; and LY-01013.

As an aspect of the invention, examples of the immune checkpoint inhibitor, which is a tubulin polymerization inhibitor, include SGN-CD48A.

According to an aspect of the invention, examples of the immune checkpoint inhibitory drug include an anti-CTLA-4 antibody (for example, Ipilimumab (YERVOY (registered trademark)) and Tremelimumab), an anti-PD-1 antibody (for example, human anti-human PD-1 monoclonal (neutralizing) antibody (for example, Nivolumab (OPDIVO (registered trademark)) and REGN-2810), a humanized anti-human PD-1 monoclonal (neutralizing) antibody (for example, Pembrolizumab (KEYTRUDA (registered trademark)), PDR-001, BGB-A317, AMP-514 (MEDI0680)), an anti-PD-Ll antibody (for example, Atezolizumab (RG7446, MPDL3280A), Avelumab (PF-06834635, MSB0010718C), Durvalumab (MEDI4736), BMS-936559), an anti-PD-L2 antibody, PD-L1 fusion protein, PD-L2 fusion protein (for example, AMP-224), an anti-Tim-3 antibody (for example, MBG453), an anti-LAG-3 antibody (for example, BMS-986016, LAG525), and an anti-KIR antibody (for example, Lirilumab).

According to an aspect of the invention, preferred examples of the immune checkpoint inhibitory drug used for the combination of the invention include an anti-CTLA-4 antibody, an anti-PD-1 antibody, an anti-PD-Ll antibody, an anti-PD-L2 antibody, PD-L1 fusion protein, and PD-L2 fusion protein. More preferred examples include an anti-CTLA-4 antibody, an anti-PD-1 antibody, an anti-PD-L1 antibody, an anti-PD-L2 antibody, PD-L1 fusion protein, and PD-L2 fusion protein. Particularly preferred examples include an anti-CTLA-4 antibody and an anti-PD-1 antibody.

According to an aspect of the invention, any one kind or an arbitrary plurality of kinds of these immune checkpoint inhibitory drugs can be used in combination with the compound used for the invention.

According to an aspect of the invention, the dose of the immune checkpoint inhibitory drug used for the combination of the invention may vary depending on the age, body weight, symptoms, therapeutic effect, method of administration, treatment time, and the like; however, the dose is adjusted so as to bring an optimal desired effect.

According to an aspect of the invention, for example, when an anti-PD-1 antibody is used, an aspect of the dose is 0.1 to 20 mg/kg of body weight. For example, when Nivolumab is used, an aspect of the dose is 0.3 to 10 mg/kg of body weight, and preferably 2 mg/kg, 3 mg/kg, or 6 mg/kg of body weight.

According to an aspect of the invention, for example, when an anti-CTLA-4 antibody is used, an aspect of the dose is 0.1 to 20 mg/kg of body weight. The dose is preferably 0.1 to 10 mg/kg of body weight, and more preferably 3 mg/kg or 10 mg/kg of body weight.

According to an aspect of the invention, above all, for example, with regard to cancer patients for whom the therapeutic effect is not sufficient when an immune checkpoint inhibitory drug is used solely, the combination of the invention can be expected to exhibit, in particular, its anti-tumor effect at the maximum. Furthermore, it is also made possible by the combination of the invention to administer each drug at a decreased dosage, and reduction of side effects can be expected.

EXAMPLES

Hereinafter, the present invention is described in more detail by way of Reference Examples, Examples, Test Examples, and Preparation Examples; however, the invention is not to be limited to these only.
The test compounds used in Test Examples 1 and 2 are as follows.
According to the description of Examples 1 to 249 of WO 2013/024898, the compounds used in Test Examples 1 and 2 were prepared and used. The activity data of the compounds used in Test Examples 1 and 2 are as described in Tables 1 to 17 of WO 2013/024898.

Test Example 1 Test for mPGES-1 inhibitory activity

A mPGES-1 microsome was prepared from CHO-K1 cells transiently transfected with a plasmid encoding human mPGES-1 cDNA. The mPGES-1 microsome was diluted in a potassium phosphate buffer solution pH 7.4 containing reduced glutathione, a DMSO solution of a test compound or DMSO solely (DMSO final concentration of 1% in both cases) was added thereto, and the mixture was incubated at 4°C for 20 minutes. Next, a solution prepared by dissolving PGH2 substrate to a final concentration of 1 μM was added to the mixture to initiate an enzymatic reaction, and the mixture was incubated at 4°C for 60 seconds. A solution of ferric chloride and a citrate (final concentrations of 1 mg/mL and 50 mM, respectively) was added to the mixture to complete the reaction. PGE2 thus formed was measured by using an HTRF kit (Cisbio International product catalogue #62P2APEC). A solution free of the test compound was used as a positive control, and a solution free of the test compound and the microsome was used as a negative control. 100% activity was defined as PGE2 production in the positive control minus PGE2 production in the negative control. Next, the IC50 value was calculated by using a standard method.

Test Example 2 Test for inhibition of PGE2 and PGF2α production in A549 cells

Human A549 cells were seeded at a rate of $2\times10^4$ cells in 100 μL/well (96-well plate), and the cells were incubated overnight. After the medium was removed, the cells were washed with a phosphate buffered saline, and then the culture medium was replaced with 3% FBS-containing RPMI medium containing a DMSO solution of a test compound or DMSO solely (DMSO final concentration of 0.1% in both cases). The cells were incubated for 60 minutes, subsequently IL-1β (5 ng/well) was added thereto, and the cells were incubated at 37°C for 24 hours. Subsequently, PGE2 in the medium was measured by using an HTRF kit (Cisbio International product catalogue #62P2APEC), and PGF2α was measured by using an EIA kit (Cayman Chemical Company product catalogue #516011). A solution free of the test compound was used as a positive control, and a solution free of the test compound and IL-1β was used as a negative control. 100% activity was defined as PGE2 and PGF2α production in the positive control minus PGE2 and PGF2α production in the negative control. Next, the IC50 value was measured by using a standard method.
The test compounds used in Test Example 3 are as follows.

Test compound 1 and test compound 2

According to the description of Example 239 and Example 89 of WO 2013/024898, compound 1: N-(3-chloro-2-methylphenyl)-2-(methoxymethyl)-6-({[2-(trifluoromethyl)phenyl]carbonyl}amino)-1H-benzimidazole-4-carboxamide 4-methylbenzenesulfonate, and compound 2: N-(3-chloro-2-methylphenyl)-2-[1-(trifluoromethyl)cyclopropyl]-6-({[2-(trifluoromethyl)phenyl]carbonyl}amino)-1H-benzimidazole-4-carboxamide were prepared and used (hereinafter, also referred to as compound 1 and compound 2).

Test Example 3: Effect of using mPGES-1 inhibitor and immune checkpoint inhibitory drug in combination in allograft model of mouse colorectal cancer cell line CT26

An effect of using mPGES-1 inhibitor (compounds 1 and 2) and an immune checkpoint inhibitory drug (anti-mouse PD-1 antibody) in combination in an allograft model of mouse colorectal cancer cell line CT26 (Cancer Res. (2013), 73(12), p3591-603) was evaluated.

1. Drug to be tested

Compound 1: N-(3-chloro-2-methylphenyl)-2-(methoxymethyl)-6-({[2-(trifluoromethyl)phenyl]carbonyl}amino)-1H-benzimidazole-4-carboxamide 4-methylbenzenesulfonate

Compound 2: N-(3-chloro-2-methylphenyl)-2-[1-(trifluoromethyl)cyclopropyl]-6-({[2-(trifluoromethyl)phenyl]carbonyl}amino)-1H-benzimidazole-4-carboxamide
Anti-mouse PD-1 antibody: InVivoMAb anti-mouse PD-1 (CD279) (Bio X Cell, Inc. (West Lebanon, NH, USA))

2. Preparation of cells for transplantation

CT26 was cultured in a $CO_2$ incubator using RPMI-1640 medium containing 10 vol% of FBS and 1 vol% of Penicillin-Streptomycin. On the day of transplantation, after the culture supernatant was removed, CT26 was washed with HBSS and collected. Collected CT26 was suspended in HBSS and used as cells for transplantation.

3. Creation of allograft model

Under anesthesia, $3.0\times10^5$ cells for transplantation were subcutaneously transplanted into the right-hand side abdomen of each female BALB/c mouse. On the seventh day of transplantation, the mice were assigned to four groups, namely, a control group a single therapy group with an mPGES-1 inhibitor (compounds 1 and 2), a single therapy group with an anti-mouse PD-1 antibody, and a combination therapy group (compound 1 or 2 and anti-mouse PD-1 antibody), each group including 4 to 6 individuals.

4. Measurement of antitumor activity

Compound 1 or 2 was repeatedly orally administered to the mice of the single therapy group with the compound 1 or 2 and the combination therapy group, at a rate of 100 mg/kg (as a free form of substance), twice a day, from the 7$^{th}$ day of transplantation to the 20$^{th}$ day of transplantation.
The anti-mouse PD-1 antibody was intraperitoneally administered to the mice of the single therapy group of the anti-mouse PD-1 antibody and the combination therapy group, at a dosage of 100 μg/individual, twice a week after transplantation, from the 7$^{th}$ day of transplantation to the 20$^{th}$ day of transplantation.
Additionally, 5% methylcellulose was repeatedly orally administered to the mice of the medium group and the single therapy group with anti-mouse PD-1 antibody during the same period as in the case of the compound 1 or 2.
Furthermore, D-PBS was intraperitoneally administered to the mice of the medium group and the single therapy group with the compound 1 or 2 during the same period as in the case of the anti-mouse PD-1 antibody.
Regarding the tumor volume ($mm^3$), the length and width of a tumor were measured by using an electronic caliper, and the tumor volume was calculated by the following formula.

[Mathematical Formula 1]

$$\text{Tumor volume} = \text{Major axis} \times (\text{minor axis})^2/2$$

Fig. 1 and Fig. 2 show the tumor volume of each group on the 21$^{st}$ day of transplantation.
The compound 1 or 2 did not suppress tumor growth when used solely, but when the compound 1 or 2 was used in combination with the anti-mouse PD-1 antibody, tumor growth was suppressed.
Test Example 4: Effect of using mPGES-1 inhibitor and immune checkpoint inhibitory drug in combination in allograft model of mouse colorectal cancer cell line CT26
An effect of using mPGES-1 inhibitor (compounds 1 and 2) and an immune checkpoint inhibitory drug (anti-mouse CTLA-4 antibody) in combination in an allograft model of mouse colorectal cancer cell line CT26 (Cancer Res. (2013), 73(12), p3591-603) was evaluated.

1. Drug to be tested

Compound 1: N-(3-chloro-2-methylphenyl)-2-(methoxymethyl)-6-({[2-(trifluoromethyl)phenyl]carbonyl}amino)-1H-benzimidazole-4-carboxamide 4-methylbenzenesulfonate
Compound 2: N-(3-chloro-2-methylphenyl)-2-[1-(trifluorometliyl)cyclopropyl]-6-({[2-(trifluoromethyl)phenyl]carbonyl}amino)-1H-benzimidazole-4-carboxamide
Anti-mouse CTLA-4 antibody: InVivoMAb anti-mouse CTLA-4 (CD152) (Bio X Cell, Inc. (West Lebanon, NH, USA))

2. Preparation of cells for transplantation

CT26 was cultured in a $CO_2$ incubator using RPMI-1640 medium containing 10 vol% of FBS and 1 vol% of Penicillin-Streptomycin. On the day of transplantation, after the culture supernatant was removed, CT26 was washed with HBSS and collected. Collected CT26 was suspended in HBSS and used as cells for transplantation.

3. Creation of allograft model

Under anesthesia, $3.0 \times 10^5$ cells for transplantation were subcutaneously transplanted into the right-hand side abdomen of each female BALB/c mouse. On the seventh day of transplantation, the mice were assigned to six groups, namely, a control group, a single therapy group with mPGES-1 inhibitor (compounds 1 and 2), a single therapy group with an anti-mouse CTLA-4 antibody, and a combination group (compound 1 or 2 and anti-mouse CTLA-4 antibody), each group including 3 individuals.

4. Measurement of antitumor activity

Compound 1 or 2 was repeatedly orally administered to the mice of the single therapy group with the compound 1 or 2 and the combination therapy group, at a rate of 100 mg/kg (as a free substance), twice a day, from the $8^{th}$ day of transplantation to the $20^{th}$ day of transplantation.

The anti-mouse CTLA-4 antibody was intraperitoneally administered to the mice of the single therapy group with the anti-mouse CTLA-4 antibody and the combination group, at a dosage of 100 μg/individual, twice a week after transplantation, from the $8^{th}$ day of transplantation to the $20^{th}$ day of transplantation.

Additionally, 5% methylcellulose was repeatedly orally administered to the mice of the medium group and the single therapy group with the anti-mouse CTLA-4 antibody during the same period as in the case of the compound 1 or 2.

Furthermore, D-PBS was intraperitoneally administered to the mice of the medium group and the single therapy group with the compound 1 or 2 during the same period as in the case of the anti-mouse CTLA-4 antibody.

Regarding the tumor volume ($mm^3$), the length and width of a tumor were measured by using an electronic caliper, and the tumor volume was calculated by the following formula.

[Mathematical Formula 2]

$$\text{Tumor volume} = \text{Major axis} \times (\text{minor axis})^2/2$$

The compound 1 or 2 did not suppress tumor growth when used solely, but when the compound 1 or 2 was used in combination with the anti-mouse CTLA-4 antibody, tumor growth was suppressed.

Preparation Example 1

Tablet (tablet for internal use)

In one 80-mg tablet of formulation,

| | |
|---|---|
| Present compound | 5.0 mg |
| Corn starch | 46.6 mg |
| Crystalline cellulose | 24.0 mg |
| Methylcellulose | 4.0 mg |
| Magnesium stearate | 0.4 mg |

A mixed powder of these proportions was tableted by a conventional method to obtain tablets for internal use.

INDUSTRIAL APPLICABILITY

The present invention relates to a prophylactic and/or therapeutic agent for cancer containing an mPGES-1 inhibitor as an active ingredient, the agent being useful in combination with an immune checkpoint inhibitor, and the

invention has industrial applicability.

## Claims

1. A prophylactic and/or therapeutic agent for cancer containing an mPGES-1 inhibitor as an active ingredient, for use in combination with an immune checkpoint inhibitor.

2. The prophylactic and/or therapeutic agent for cancer according to claim 1, wherein the mPGES-1 inhibitor is a compound of Formula [1]:

[Chemical Formula 1]

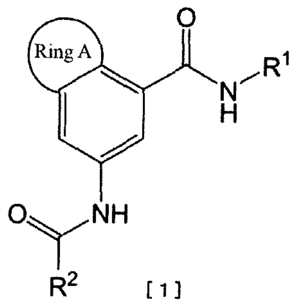

[1]

or a tautomer of the compound, or a pharmaceutically acceptable salt thereof wherein ring A represents a group represented by Formula [2], [3], or [4]:

[Chemical Formula 2]

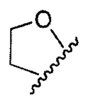

[2]

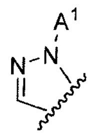

[3]

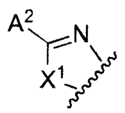

[4]

wherein

$X^1$ represents NH, N-alkyl, or O;
$A^1$ represents hydrogen or alkyl;
$A^2$ represents:

i) hydrogen,
ii) halogen,
iii) alkyl which may be substituted with one to three groups selected from the group consisting of halogen, amino, monoalkylamino, dialkylamino, carbamoyl, monoalkylaminocarbonyl, dialkylaminocarbonyl, saturated cyclic aminocarbonyl, alkoxy, alkoxyalkoxy, and alkylcarbonyloxy,
iv) cycloalkyl which may be substituted with alkyl, wherein said alkyl may be substituted with one to three halogens,
v) alkoxy,
vi) a saturated heterocyclic group which may be substituted with alkyl, alkyloxycarbonyl, alkylcarbonyl, or oxo,
vii) alkylthio,
viii) alkylsulfonyl,
ix) alkylsulfinyl,
x) Formula [5]:

[Chemical Formula 3]

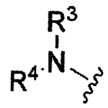

[5]

wherein $R^3$ and $R^4$ are identical or different and each represent:

a) hydrogen,
b) alkyl which may be substituted with a group selected from the group consisting of monoalkylamino, dialkylamino, saturated cyclic amino optionally substituted with alkyl, a saturated heterocyclic group optionally substituted with alkyl, alkoxy, hydroxycarbonyl, hydroxyl, alkyloxycarbonyl, and alkylthio, or
c) cycloalkyl,
or

xi) saturated cyclic amino which may be substituted with alkyl, amino, monoalkylamino, dialkylamino, alkoxy, or hydroxyl;

$R^1$ represents phenyl, benzyl, naphthyl, cycloalkyl, cycloalkylmethyl, heteroaryl, heteroarylmethyl, 1,2,3,4-tetrahydronaphthalen-5-yl, 1,2,3,4-tetrahydronaphthalen-6-yl, 2,3-dihydro-1H-inden-4-yl, 2,3-dihydro-1H-inden-5-yl, 1,2-dihydrocyclobutabenzen-3-yl, 1,2-dihydrocyclobutabenzen-4-yl, or alkyl, wherein said phenyl, benzyl, cycloalkyl, cycloalkylmethyl, heteroaryl, and heteroarylmethyl may be substituted with one to three groups selected from the group consisting of:

i) halogen,
ii) alkyl which may be substituted with one to three groups selected from the group consisting of halogen, hydroxy, and phenyl,
iii) alkoxy,
iv) hydroxy, and
v) cyano;

$R^2$ represents phenyl or pyridyl, wherein said phenyl and pyridyl may be substituted with one to three groups selected from the group consisting of:

i) halogen,
ii) alkylsulfonyl,
iii) alkoxy which may be substituted with one to three halogens or alkoxies,
iv) alkynyl which may be substituted with alkoxyalkyl or cycloalkyl, and
v) alkyl which may be substituted with one to three groups selected from the group consisting of alkoxy, alkoxyalkoxy, cycloalkyl, phenyl, and halogen.

**3.** The prophylactic and/or therapeutic agent for cancer according to claim 2,
wherein the ring A represents a group represented by Formula [4], and $X^1$ represents NH.

**4.** The prophylactic and/or therapeutic agent for cancer according to claim 2 or 3,
wherein $R^1$ represents phenyl, 1,2,3,4-tetrahydronaphthalen-5-yl, 1,2,3,4-tetrahydronaphthalen-6-yl, 2,3-dihydro-1H-inden-4-yl, 2,3-dihydro-1H-inden-5-yl, 1,2-dihydrocyclobutabenzen-3-yl, or 1,2-dihydrocyclobutabenzen-4-yl, wherein said phenyl may be substituted with one to three groups selected from the group consisting of:

i) halogen,
ii) alkyl which may be substituted with one to three halogens,
iii) alkoxy, and
iv) cyano.

**5.** The prophylactic and/or therapeutic agent for cancer according to any one of claims 2 to 4,

wherein $R^2$ represents phenyl, and
said phenyl may be substituted with one to three groups selected from the group consisting of:

i) halogen,
ii) alkylsulfonyl,
iii) alkoxy which may be substituted with alkoxy,
iv) alkynyl which may be substituted with alkoxyalkyl or cycloalkyl, and
v) alkyl which may be substituted with one to three groups selected from the group consisting of halogen, alkoxy, alkoxyalkoxy, cycloalkyl, and phenyl.

**6.** The prophylactic and/or therapeutic agent for cancer according to any one of claims 2 to 5,

wherein the ring A represents a group represented by General Formula [4];
$X^1$ represents NH;
$A^2$ represents:

i) hydrogen,
ii) alkyl which may be substituted with a group selected from the group consisting of halogen, monoalkylamino, dialkylamino, monoalkylaminocarbonyl, dialkylaminocarbonyl, saturated cyclic aminocarbonyl, alkoxy, alkoxyalkoxy, and alkylcarbonyloxy,
iii) cycloalkyl which may be substituted with alkyl optionally substituted with one to three halogens,
iv) alkoxy,
v) a saturated heterocyclic group which may be substituted with alkyl or alkyloxycarbonyl,
vi) alkylthio,
vii) alkylsulfonyl,
viii) alkylsulfinyl,
ix) amino substituted with alkyl which may be substituted with a group selected from the group consisting of monoalkylamino, dialkylamino, saturated cyclic amino optionally substituted with alkyl, tetrahydrofuryl, morpholino, alkoxy, hydroxycarbonyl, hydroxyl, and alkylthio,
x) amino substituted with cycloalkyl, or
xi) saturated cyclic amino which may be substituted with alkyl, dialkylamino, alkoxy, or hydroxyl;

$R^1$ represents:

i) phenyl which may be substituted with one to three groups selected from the group consisting of halogen, alkyl optionally substituted with one to three halogens, alkoxy, and cyano,
ii) 1,2,3,4-tetrahydronaphthalen-5-yl,
iii) 2,3-dihydro-1H-inden-5-yl,
iv) benzyl which may be substituted with halogen or with alkyl optionally substituted with one to three halogens,
v) cycloalkyl,
vi) cycloalkylmethyl,
vii) naphthyl,
viii) pyridylmethyl which may be substituted with alkyl optionally substituted with one to three halogens,
ix) thienyl,
x) thienylmethyl,
xi) benzothiazolyl,
xii) benzothiadiazolyl,
xiii) indolyl, or
xiv) alkyl; and

$R^2$ represents phenyl or pyridyl,

wherein said phenyl may be substituted with one to three groups selected from the group consisting of:

i) halogen,
ii) alkylsulfonyl,
iii) alkoxy which may be substituted with alkoxy,

iv) alkynyl which may be substituted with alkoxyalkyl or cycloalkyl, and
v) alkyl which may be substituted with one to three groups selected from the group consisting of halogen, alkoxy, alkoxyalkoxy, cycloalkyl, and phenyl, and

said pyridyl may be substituted with halogen.

**7.** The prophylactic and/or therapeutic agent for cancer according to any one of claims 2 to 6,

wherein the ring A represents a group represented by General Formula [4];
$X^1$ represents NH;
$A^2$ represents alkoxy-substituted alkyl, dialkylamino, tetrahydrofuryl, tetrahydrofurylmethyl, alkoxyalkylamino, or cycloalkyl which may be substituted with unsubstituted alkyl or alkyl substituted with one to three halogens;
$R^1$ represents phenyl substituted with one halogen and one methyl; and
$R^2$ represents phenyl which may be substituted with one trifluoromethyl or two halogens.

**8.** The prophylactic and/or therapeutic agent for cancer according to any one of claims 2 to 7, wherein the mPGES-1 inhibitor is a compound selected from the group consisting of:

(1) N-[2-(trifluoromethyl)benzyl]-6-({[2-(trifluoromethyl)phenyl]carbonyl} amino)-1H-benzimidazole-4-carboxamide,
(2) N-cyclohexyl-6-({[2-(trifluoromethyl)phenyl]carbonyl}amino)-1H-benzimidazole-4-carboxamide,
(3) N-(3-chloro-2-methylphenyl)-6-({[2-(trifluoromethyl)phenyl]carbonyl}amino)-1H-benzimidazole-4-carboxamide,
(4) N-[(1-hydroxycyclohexyl)methyl]-6-({[2-(trifluoromethyl)phenyl]carbonyl}amino)-1H-benzimidazole-4-carboxamide,
(5) N-[2-(trifluoromethyl)benzyl]-5-({[2-(trifluoromethyl)phenyl]carbonyl} amino}-2,3-dihydro-1-benzofuran-7-carboxamide,
(6) N-cyclohexyl-5-({[2-(trifluoromethyl)phenyl]carbonyl}amino)-2,3-dihydro-1-benzofuran-7-carboxamide,
(7) N-(3-chloro-2-methylphenyl)-5-({[2-(trifluoromethyl)phenyl]carbonyl} amino)-2,3-dihydro-1-benzofuran-7-carboxamide,
(8) N-cyclohexyl-5-({[2-(trifluoromethyl)phenyl]carbonyl}amino)-1H-indazole-7-carboxamide,
(9) N-[2-(trifhioromethyl)benzyl]-5-({[2-(trifluoromethyl)phenyl]carbonyl} amino)-1H-indazole-7-carboxamide,
(10) N-(3-chloro-2-methylphenyl)-2-(methoxymethyl)-6-({[2-(trifluoromethyl)phenyl]carbonyl}amino)-1H-benzimidazole-4-carboxamide,
(11) 2-methyl-N-[2-(trifluoromethyl)benzyl]-6-({[2-(trifluoromethyl)phenyl]carbonyl} amino)-1H-benzimidazole-4-carboxamide,
(12) N-cyclohexyl-2-methyl-6-({[2-(trifluoromethyl)phenyl]carbonyl}amino)-1H-benzimidazole-4-carboxamide,
(13) N-(3-chloro-2-methylphenyl)-2-methyl-6-({[2-(trifluoromethyl)phenyl]carbonyl} amino)-1H-benzimidazole-4-carboxamide,
(14) N-cyclopentyl-2-methyl-6-({[2-(trifluoromethyl)phenyl] carbonyl} amino)- 1H-benzimidazole-4-carboxamide,
(15) N-cyclobutyl-2-methyl-6-({[2-(trifluoromethyl)phenyl]carbonyl}amino)-1H-benzimidazole-4-carboxamide,
(16) N-(3-chloro-2-methylphenyl)-2-ethyl-6-({[2-(trifluoromethyl)phenyl] carbonyl} amino)-1H-benzimidazole-4-carboxamide,
(17) N-cyclohexyl-2-ethyl-6-({[2-(trifluoromethyl)phenyl]carbonyl}amino)-1H-benzimidazole-4-carboxamide,
(18) 2-ethyl-N-[2-(trifluoromethyl)benzyl]-6-({[2-(trifluoromethyl)plienyl]carbonyl}amino)-1H-benzimidazole-4-carboxamide,
(19) N-cyclohexyl-2-(methoxymethyl)-6-({[2-(trifluoromethyl)phenyl]carbonyl} amino)-1H-benzimidazole-4-carboxamide,
(20) 2-(methoxymethyl)-N-[2-(trifluoromethyl)benzyl]-6-({[2-(trifluoromethyl)phenyl]carbonyl}amino)-1H-benzimidazole-4-carboxamide,
(21) 2-(methoxymethyl)-N-(2-methylphenyl)-6-({[2-(trifluoromethyl)phenyl]carbonyl}amino)-1H-benzimidazole-4-carboxamide,
(22) 2-(methoxymethyl)-N-(4-methylphenyl)-6-({[2-(trifluoromethyl)phenyl]carbonyl}amino)-1H-benzimidazole-4-carboxamide,
(23) N-(2-chlorobenzyl)-2-(methoxymethyl)-6-({[2-(trifluoromethyl)phenyl]carbonyl}amino)-1H-benzimidazole-4-carboxamide,
(24) 2-(methoxymethyl)-N-(4-methylbenzyl)-6-({[2-(trifluoromethyl)phenyl]carbonyl}amino)-1H-benzimidazole-4-carboxamide,
(25) N-(4,4-difluorocyclohexyl)-2-(methoxymethyl)-6-({[2-(trifluoromethyl)phenyl]carbonyl}amino)-1H-benzimidazole-4-carboxamide,
(26) N-(4-tert-butylphenyl)-2-(methoxymethyl)-6-({[2-(trifluoromethyl)phenyl)carbonyl}amino)-1H-benzimidazole-4-carboxamide,
(27) 2-(methoxymethyl)-N-[4-(trifluoromethyl)phenyl]-6-({[2-(trifluoromethyl)phenyl] carbonyl}amino)-1H-benzimidazole-4-carboxamide,
(28) N-(2,4-dimethylphenyl)-2-(methoxymethyl)-6-({[2-(trifluoromethyl)phenyl] carbonyl} amino)-1H-benzimidazole-4-carboxamide,
(29) N-(2-chloro-4-methylphenyl)-2-(methoxymethyl)-6-({[2-(trifluoromethyl)phenyl]carbonyl} amino)-1H-benzimidazole-4-carboxamide,
(30) N-(3,4-dimethylphenyl)-2-(methoxymethyl)-6-({[2-(trifluoromethyl)phenyl]carbonyl}amino)-1H-benzimidazole-4-carboxamide,
(31) N-(3-chloro-4-methylphenyl)-2-(methoxymethyl)-6-({[2-(trifluoromethyl)phenyl]carbonyl}amino)-1H-benzimidazole-4-carboxamide,
(32) N-(2,3-dihydro-1H-inden-5-yl)-2-(methoxymethyl)-6-({[2-(trifluoromethyl)phenyl]carbonyl} amino)-1H-benzimidazole-4-carboxamide,
(33) 2-(methoxymethyl)-N-(5,6,7,8-tetrahydronaphthalen-1-yl)-6-({[2-(trifluoromethyl)phenyl]carbonyl}amino)-1H-benzimidazole-4-carboxamide,
(34) N-(2-fluorophenyl)-2-(methoxymethyl)-6-({[2-(trifluoromethyl)phenyl]carbonyl}amino)-1H-benzimidazole-4-carboxamide,
(35) 2-(methoxymethyl)-N-(2-methoxyphenyl)-6-({[2-(trifluoromethyl)phenyl]carbonyl}amino)-1H-benzimidazole-4-carboxamide,
(36) 2-(methoxymethyl)-N-(4-methoxyphenyl)-6-({[2-(trifluoromethyl)phenyl]carbonyl}amino)-1H-benzimidazole-4-carboxamide,
(37) N-(3-bromo-2-methylphenyl)-2-(methoxymethyl)-6-({[2-(trifluoromethyl)phenyl]carbonyl}amino)-1H-benzimidazole-4-carboxamide,
(38) N-(3-chloro-2-methylbenzyl)-2-(methoxymethyl)-6-({[2-(trifluoromethyl)phenyl]carbonyl}amino)-1H-benzimidazole-4-carboxamide,
(39) N-(2,6-difluorophenyl)-2-(methoxymethyl)-6-({[2-(trifluoromethyl)phenyl]carbonyl}amino)-1H-benzimidazole-4-carboxamide,
(40) N-(3-cyano-2-methylphenyl)-2-(methoxymethyl)-6-({[2-(trifluoromethyl)phenyl]carbonyl} amino)-1H-benzimidazole-4-carboxamide,
(41) 2-(methoxymethyl)-6-({[2-(trifluoromethyl)phenyl]carbonyl}amino)-N-{[3-(trifluoromethyl)pyridin-2-yl]methyl} -1H-benzimidazole-4-carboxamide,
(42) N-(2-chloro-6-methylphenyl)-2-(methoxymethyl)-6-({[2-(trifluoromethyl)phenyl]carbonyl} amino)-1H-benzimidazole-4-carboxamide,
(43) 2-(2-amino-2-oxoethyl)-N-(3-chloro-2-methylphenyl)-6-({[2-(trifluoromethyl)phenyl]carbonyl}amino)-1H-benzimidazole-4-carboxamide,
(44) 2-(2-amino-2-oxoethyl)-N-[2-(trifluoromethyl)benzyl]-6-({[2-(trifluoromethyl)phenyl]carbonyl}amino)-1H-benzimidazole-4-carboxamide,
(45) N-(3-chloro-2-methylphenyl)-1-methyl-6-({[2-(trifluoromethyl)phenyl]carbonyl}amino)-1H-benzimidazole-4-carboxamide,
(46) N-cyclohexyl-1-methyl-6-({[2-(trifluoromethyl)phenyl]carbonyl}amino)-1H-benzimidazole-4-carboxamide,
(47) 1-metliyl-N-[2-(trifluoromethyl)benzyl]-6-({[2-(trifluoromethyl)phenyl]carbonyl}amino)-1H-benzimidazole-4-carboxamide,
(48) N-(3-chloro-2-methylphenyl)-1-ethyl-6-({[2-(trifluoromethyl)phenyl]carbonyl}amino)-1H-benzimidazole-4-carboxamide,
(49) N-cyclohexyl-1-ethyl-6-({[2-(trifluoromethyl)phenyl]carbonyl}amino)-1H-benzimidazole-4-carboxamide,
(50) 1-ethyl-N-[2-(trifluoromethyl)benzyl]-6-({[2-(trifluoromethyl)phenyl]carbonyl}amino)-1H-benzimidazole-4-carboxamide,
(51) N-(3-chloro-2-methylphenyl)-2-methyl-6-({[2-(trifluoromethyl)phenyl]carbonyl}amino)-1,3-benzoxazole-4-carboxamide,
(52) 2-methyl-N-[2-(trifluoromethyl)benzyl]-6-({[2-(trifluoromethyl)phenyl]carbonyl} amino)-1,3-benzoxazole-4-carboxamide,
(53) N-(3-chloro-2-methylphenyl)-2-ethyl-6-({[2-(trifluoromethyl)phenyl]carbonyl} amino)-1,3-benzoxazole-4-carboxamide,
(54) N-(3-chloro-2-methylphenyl)-2-ethoxy-6-({[2-(trifluoromethyl)phenyl]carbonyl}amino)-1H-benzimidazole-4-carboxamide,
(55) 2-ethoxy-N-[2-(trifluoromethyl)benzyl]-6-({[2-(trifluoromethyl)phenyl]carbonyl}amino)-1H-benzimidazole-4-carboxamide,
(56) N-(3-chloro-2-methylphenyl)-2-(1-chloro-2-methylpropan-2-yl)-6-({[2-(trifluoromethyl)phenyl]carbonyl}amino)-1H-benzimidazole-4-carboxamide,
(57) N-(3-chloro-2-methylphenyl)-2-[(dimethylamino)methyl]-6-({[2-(trifluoromethyl)phenyl]carbonyl}amino)-1H-benzimidazole-4-carboxamide,
(58) N-(3-chloro-2-methylphenyl)-2-(2-methylpropyl)-6-({[2-(trifluoromethyl)phenyl]carbonyl}amino)-1H-benzimidazole-4-carboxamide,
(59) 2-(2-methylpropyl)-N-[2-(trifluoromethyl)benzyl]-6-({[2-(trifluoromethyl)phenyl]carbonyl}amino)-1H-benzimidazole-4-carboxamide,
(60)tert-butyl3-{4-[(3-chloro-2-methylphenyl)carbamoyl]-6-({[2-(trifluoromethyl)phenyl]carbonyl}amino)-1H-benzimidazol-2-yl}azetidine-1-carboxylate,
(61) N-(3-chloro-2-methylphenyl)-2-[(methylamino)methyl]-6-({[2-(trifluoromethyl)phenyl]carbonyl}amino)-1H-benzimidazole-4-carboxamide,
(62) {4-[(3-chloro-2-methylphenyl)carbamoyl]-6-({[2-(trifluoromethyl)phenyl]carbonyl}amino)-1H-benzimidazol-2-yl}methyl acetate,
(63) N-(3-chloro-2-methylphenyl)-2-[(2R)-tetrahydrofuran-2-yl]-6-({[2-(trifluoromethyl)phenyl]carbonyl}amino)-1H-benzimidazole-4-carboxamide,
(64) 2-[(2R)-tetrahydrofuran-2-yl]-N-[2-(trifluoromethyl)benzyl]-6-({[2-(trifluoromethyl)phenyl]carbonyl}amino)-1H-benzimidazole-4-carboxamide,
(65) N-(3-chloro-2-methylphenyl)-2-[(2S)-tetrahydrofuran-2-yl]-6-({[2-(trifluoromethyl)phenyl]carbonyl}amino)-1H-benzimidazole-4-carboxamide,
(66) 2-[(2S)-tetrahydrofuran-2-yl]-N-[2-(trifluoromethyl)benzyl]-6-({[2-(trifluoromethyl)phenyl]carbonyl}amino)-1H-benzimidazole-4-carboxamide,
(67) 2-(1-acetylazetidin-3-yl)-N-(3-chloro-2-methylphenyl)-6-({[2-(trifluoromethyl)phenyl]carbonyl} amino)-1H-benzimidazole-4-carboxamide,
(68) tert-butyl (2S)-2-{4-[(3-chloro-2-methylphenyl)carbamoyl]-6-({[2-(trifluoromethyl)phenyl]carbonyl}amino)-1H-benzimidazol-2-yl}pyrrolidine-1-carboxylate,
(69) tert-butyl (2R)-2-{4-[(3-chloro-2-methylphenyl)carbamoyl]-6-({[2-(trifluoromethyl)phenyl]carbonyl)amino)-1H-benzimidazol-2-yl}pyrrolidine-1-carboxylate,
(70) N-(3-chloro-2-methylphenyl)-2-[(2S)-pyrrolidin-2-yl]-6-({[2-(trifluoromethyl)phenyl]carbonyl} amino)-1H-benzimidazole-4-carboxamide,
(71) N-(3-chloro-2-methylphenyl)-2-[(2S)-1-methylpyrrolidin-2-yl]-6-({[2-(trifluoromethyl)phenyl]carbonyl} amino)-1H-benzimidazole-4-carboxamide,
(72) 2-[(2S)-1-acetylpyrrolidin-2-yl]-N-(3-chloro-2-methylphenyl)-6-({[2-(trifluoromethyl)phenyl]carbonyl}amino)-1H-benzimidazole-4-carboxamide,
(73) N-(3-chloro-2-methylphenyl)-2-[(2-methoxyethoxy)methyl]-6-({[2-(trifluoromethyl)phenyl]carbonyl}amino)-1H-benzimidazole-4-carboxamide,
(74) N-(3-chloro-2-methylphenyl)-2-(1-methoxy-2-methylpropan-2-yl)-6-({[2-(trifluoromethyl)phenyl]carbonyl}amino)-1H-benzimidazole-4-carboxamide,
(75) 2-tert-butyl-N-(3-chloro-2-methylphenyl)-6-({[2-(trifluoromethyl)phenyl]carbonyl}amino)-1H-benzimidazole-4-carboxamide,
(76) 2-tert-butyl-6-({[2-(trifluoromethyl)phenyl]carbonyl}amino)-N-{[3-(trifluoromethyl)pyridin-2-yl]methyl}-1H-benzimidazole-4-carboxamide,
(77) N-(3-chloro-2-methylphenyl)-2-(2-ethoxyethyl)-6-({[2-(trifluoromethyl)phenyl]carbonyl}amino)-1H-benzimidazole-4-carboxamide,
(78) N-(3-chloro-2-methylphenyl)-2-(ethoxymethyl)-6-({[2-(trifluoromethyl)phenyl]carbonyl} amino)-1H-benzimidazole-4-carboxamide,
(79) 2-(ethoxymethyl)-6-({[2-(trifluoromethyl)phenyl]carbonyl}amino)-N- {[3-(trifluoromethyl)pyridin-2-yl]methyl}-1H-benzimidazole-4-carboxamide,
(80) N-(3-chloro-2-methylphenyl)-2-(2-methoxyethyl)-6-({[2-(trifluoromethyl)phenyl]carbonyl}amino)-1H-benzimidazole-4-carboxamide,
(81) N-(3-chloro-2-methylphenyl)-2-(2,2-dimethylpropyl)-6-({[2-(trifluoromethyl)phenyl]carbonyl}amino)-1H-benzimidazole-4-carboxamide,
(82) N-(3-chloro-2-methylphenyl)-2-cyclopropyl-6-({[2-(trifluoromethyl)phenyl]carbonyl}amino)-1H-benzimidazole-4-carboxamide,
(83) N-(3-chloro-2-methylphenyl)-2-(2-methylpentan-2-yl)-6-({[2-(trifluoromethyl)phenyl]carbonyl)amino)-1H-benzimidazole-4-carboxamide,
(84) N-(3-chloro-2-methylphenyl)-2-(1-methylcyclopropyl)-6-({[2-(trifluoromethyl)phenyl]carbonyl}amino)-1H-benzimidazole-4-carboxamide,
(85) 2-tert-butyl-N-(3-chloro-4-methylphenyl)-6-({[2-(trifluoromethyl)phenyl]carbonyl}amino)-1H-benzimidazole-4-carboxamide,
(86) 2-tert-butyl-N-(3-chloro-2-methylphenyl)-6- {[(2,5-dichlorophenyl)carbonyl]amino}-1H-benzimidazole-4-carboxamide,
(87) 2-tert-butyl-N-(3-chloro-4-methylphenyl)-6-{[(2,5-dichlorophenyl)carbonyl]amino}-1H-benzimidazole-4-carboxamide,
(88) N-(3-chloro-2-methylphenyl)-2-[1-(trifluoromethyl)cyclopropyl]-6-({[2-(trifluoromethyl)phenyl]carbonyl}amino)-1H-benzimidazole-4-carboxamide,
(89) N-(3-chloro-2-methylphenyl)-2-(methoxymethyl)-1-methyl-6-({[2-(trifluoromethyl)phenyl]carbonyl}amino)-1H-benzimidazole-4-carboxamide,
(90) N-(2-chlorobenzyl)-2-(methoxymethyl)-1-methyl-6-({[2-(trifluoromethyl)phenyl]carbonyl}amino)-1H-benzimidazole-4-carboxamide,
(91) 6-{[(2-chloro-6-fluorophenyl)carbonyl]amino}-N-(3-chloro-2-methylphenyl)-2-(methoxymethyl)-1H-benzimidazole-4-carboxamide,
(92) 6- {[(2-chloro-4-fluorophenyl)carbonyl]amino}-N-(3-chloro-2-methylphenyl)-2-methoxymethyl-1H-benzimidazole-4-carboxamide,
(93) 6-{[(2-chloro-5-fluorophenyl)carbonyl]amino}-N-(3-chloro-2-methylphenyl)-2-(methoxymethyl)-1H-benzimidazole-4-carboxamide,
(94) N-(3-chloro-2-methylphenyl)-6-{[(2-chlorophenyl)carbonyl]amino}-2-(methoxymethyl)-1H-benzimidazole-4-carboxamide,
(95) N-(3 -chloro-2-methylphenyl)-6-{[(2-chloropyridin-3-yl)carbonyl]amino} -2-(methoxymethyl)-1H-benzimidazole-4-carboxamide,
(96) 6-{[(2-bromophenyl)carbonyl]amino}-N-(3-chloro-2-methylphenyl)-2-(methoxymethyl)-1H-benzimidazole-4-carboxamide,
(97) N-(3-chloro-2-methylphenyl)-6-{[(2,6-dichlorophenyl)carbonyl]amino}-2-(methoxymethyl)-1H-benzimidazole-4-carboxamide,
(98) N-(3-chloro-2-methylphenyl)-6-{[(2,5-dichlorophenyl)carbonyl]amino} -2-(methoxymethyl)-1H-benzimidazole-4-carboxamide,
(99) 6-{[(2-chloro-3-fluorophenyl)carbonyl]amino}-N-(3-chloro-2-meth ylphenyl) - 2-(methoxymethyl)-1H-benzimidazole-4-carboxamide,
(100) 6-{[(2-chloro-3,6-difluorophenyl)carbonyl]amino}-N-(3-chloro-2-methylphenyl)-2-(methoxymethyl)-1H-benzimidazole-4-carboxamide,
(101) 6-{[(2-bromo-6-chlorophenyl)carbonyl]amino}-N-(3-chloro-2-methylphenyl)-2-(methoxymethyl)-1H-benzimidazole-4-carboxamide,
(102) 6-{[(2-bromo-6-fluorophenyl)carbonyl]amino}-N-(3-chloro-2-methylphenyl)-2-(methoxymethyl)-1H-benzimidazole-4-carboxamide,
(103) N-(3-chloro-2-methylphenyl)-6-{[(2-chloro-6-methylphenyl)carbonyl]amino}-2-(methoxymethyl)-1H-benzimidazole-4-carboxamide,
(104) N-(3-chloro-2-methylphenyl)-6-{[(2-chloro-4-methylphenyl)carbonyl]amino}-2-(methoxymethyl)-1H-benzimidazole-4-carboxamide,
(105) 6-{[(5-bromo-2-chlorophenyl)carbonyl]amino} -N-(3-chloro-2-methylphenyl)-2-(methoxymethyl)-1H-benzimidazole-4-carboxamide,
(106) 6-{[(2-bromo-5-chlorophenyl)carbonyl]amino}-N-(3-chloro-2-methylphenyl)-2-(methoxymethyl)-1H-benzimidazole-4-carboxamide,
(107) N-(3-chloro-2-methylphenyl)-6-{[(2-chloro-5-methylphenyl)carbonyl]amino}-2-(methoxymethyl)-1H-benzimidazole-4-carboxamide,
(108) N-(3-chloro-2-methylphenyl)-2-(methoxymethyl)-6-( {[5-methyl-2-(trifluoromethyl)phenyl]carbonyl}amino)-1H-benzimidazole-4-carboxamide,
(109) 6-({[2,5-bis(trifluoromethyl)phenyl]carbonyl}amino)-N-(3-chloro-2-methylphenyl)-2-(methoxymethyl)-1H-benzimidazole-4-carboxamide,
(110) 6-({[2,4-bis(trifluoromethyl)phenyl]carbonyl}amino)-N-(3-chloro-2-methylphenyl)-2-(methoxymethyl)-1H-benzimidazole-4-carboxamide,
(111) N-(3-chloro-2-methylphenyl)-6-({[5-fluoro-2-(trifluoromethyl)phenyl]carbonyl}amino)-2-(methoxymethyl)-1H-benzimidazole-4-carboxamide,
(112) N-(3-chloro-2-methylphenyl)-6-({[2-chloro-6-(trifluoromethyl)phenyl]carbonyl}amino)-2-(methoxymethyl)-1H-benzimidazole-4-carboxamide,
(113) N-(3-chloro-2-methylphenyl)-6-[({2-chloro-5-[2-(propan-2-yloxy)ethoxy]phenyl}carbonyl)amino]-2-(methoxymethyl)-1H-benzimidazole-4-carboxamide,
(114) 6-({[2-chloro-5-(2-ethoxyethoxy)phenyl)carbonyl} amino)-N-(3-chloro-2-methylphenyl)-2-(methoxymethyl)-1H-benzimidazole-4-carboxamide,
(115) 6-({[2-chloro-5-(3-methoxypropyl)phenyl]carbonyl}amino)-N-(3-chloro-2-methylphenyl)-2-(methoxymethyl)-1H-benzimidazole-4-carboxamide,
(116) 6-({[5-(3-tert-butoxyprop-1-yn-1-yl)-2-chlorophenyl]carbonyl}amino)-N-(3 - chloro-2-methylphenyl)-2-(methoxymethyl)-1H-benzimidazole-4-carboxamide,
(117) 6-({[5-(3-tert-butoxypropyl)-2-chlorophenyl]carbonyl}amino)-N-(3-chloro-2-methylphenyl)-2-(methoxymethyl)-1H-benzimidazole-4-carboxamide,
(118) 6-({[2-chloro-5-(3-hydroxy-3-methylbutyl)phenyl]carbonyl}amino)-N-(3-chloro-2-methylphenyl)-2-(methoxymethyl)-1H-benzimidazole-4-carboxamide,
(119) 6-({[2-chloro-5-(ethoxymethyl)phenyl]carbonyl}amino)-N-(3-chloro-2-methylphenyl)-2-(methoxymethyl)-1H-benzimidazole-4-carboxamide,
(120) 6-[({2-chloro-5-[(2-ethoxyethoxy)methyl]phenyl}carbonyl)amino]-N-(3-chloro-2-methylphenyl)-2-(methoxymethyl)-1H-benzimidazole-4-carboxamide,
(121) 6-({[2-chloro-5-(2-cyclopropylethyl)phenyl]carbonyl}amino)-N-(3-chloro-2-methylphenyl)-2-(methoxymethyl)-1H-benzimidazole-4-carboxamide,
(122) N-(3-chloro-2-methylphenyl)-6-({[2-chloro-5-(2-phenylethyl)phenyl]carbonyl} amino)-2-(methoxymethyl)-1H-benzimidazole-4-carboxamide,
(123) N-(3-chloro-2-methylphenyl)-2-cyclopentyl-6-({[2-(trifluoromethyl)phenyl]carbonyl}amino)-1H-benzimidazole-4-carboxamide,
(124) N-(3-chloro-2-methylphenyl)-2-cyclopentyl-6-{[(2,5-dichlorophenyl)carbonyl]amino} -1H-benzimidazole-4-carboxamide,
(125) 6-{[(2-chloro-6-fluorophenyl)carbonyl]amino}-N-(3-chloro-2-methylphenyl)-2-cyclopentyl-1H-benzimidazole-4-carboxamide,
(126) 6-{[(2-chloro-6-fluorophenyl)carbonyl]amino}-N-(3-chloro-2-methylphenyl)-2-[(2R)-tetrahydrofuran-2-yl]-1H-benzimidazole-4-carboxamide,
(127) N-(3-chloro-2-methylphenyl)-6-{[(2,6-dichlorophenyl)carbonyl]amino } -2-[(2R)-tetrahydrofuran-2-yl]-1H-benzimidazole-4-carboxamide,
(128) N-(3-chloro-2-methylphenyl)-6-{[(2,5-dichlorophenyl)carbonyl]amino}-2-[(2R)-tetrahydrofuran-2-yl]-1H-benzimidazole-4-carboxamide,
(129) N-(3-chloro-2-methylphenyl)-2-[(2S)-5-oxopyrrolidin-2-yl]-6-({[2-(trifluoromethyl)phenyl]carbonyl}amino)-1H-benzimidazole-4-carboxamide,
(130) N-(3-chloro-2-methylphenyl)-2-[(2R)-5-oxopyrrolidin-2-yl]-6-({[2-(trifluoromethyl)phenyl)carbonyl}amino)-1H-benzimidazole-4-carboxamide,
(131) N-(3-chloro-2-methylphenyl)-2-[2-oxo-2-(pyrrolidin-1-yl)ethyl]-6-({[2-(trifluoromethyl)phenyl] carbonyl}amino)-1H-benzimidazole-4-carboxamide,
(132) N-(3-chloro-2-methylphenyl)-2-[2-(dimethylamino)-2-oxoethyl]-6-({[2-(trifluoromethyl)phenyl]carbonyl}amino)-1H-benzimidazole-4-carboxamide,
(133) N-(3-chloro-2-methylphenyl)-2-[2-(methylamino)-2-oxoethyl]-6-({[2-(trifluoromethyl)phenyl]carbonyl}amino)-1H-benzimidazole-4-carboxamide,
(134) 2-chloro-N-(3-chloro-2-methylphenyl)-6-({[2-(trifluoromethyl)phenyl]carbonyl}amino)-1H-benzimidazole-4-carboxamide,
(135) N-(3-chloro-2-methylphenyl)-2-[(2-methoxyethyl)amino]-6-({[2-(trifluoromethyl)phenyl]carbonyl} amino)-1H-benzimidazole-4-carboxamide,
(136) N-(3-chloro-2-methylphenyl)-2-[(2-hydroxyethyl)amino]-6-({[2-(trifluoromethyl)phenyl]carbonyl}amino)-1H-benzimidazole-4-carboxamide,
(137) N-(3-chloro-2-methylphenyl)-2-(methylamino)-6-({[2-(trifluoromethyl)phenyl]carbonyl}amino)-1H-benzimidazole-4-carboxamide,
(138) N-(3-chloro-2-methylphenyl)-2-(ethylamino)-6-({[2-(trifluoromethyl)phenyl]carbonyl}amino)-1H-benzimidazole-4-carboxamide,
(139) N-(3-chloro-2-methylphenyl)-2-[(2,2-dimethylpropyl)amino]-6-({[2-(trifluoromethyl)phenyl]carbonyl} amino)-1H-benzimidazole-4-carboxamide,
(140) N-(3-chloro-2-methylphenyl)-2-(cyclopentylamino)-6-({[2-(trifluoromethyl)phenyl]carbonyl} amino)-1H-benzimidazole-4-carboxamide,
(141) N-(3-chloro-2-methylphenyl)-2-(piperidin-1-yl)-6-({[2-(trifluoromethyl)phenyl]carbonyl}amino)-1H-benzimidazole-4-carboxamide,
(142) N-(3-chloro-2-methylphenyl)-2-(4-methylpiperazin-1-yl)-6-({[2-(trifluoromethyl)phenyl]carbonyl} amino)-1H-benzimidazole-4-carboxamide,
(143) 2-[bis(2-hydroxyethyl)amino]-N-(3-chloro-2-methylphenyl)-6-({[2-(trifluoromethyl)phenyl]carbonyl}amino)-1H-benzimidazole-4-carboxamide,
(144) N-(3-chloro-2-methylphenyl)-2-(dimethylamino)-6-({[2-(trifluoromethyl)phenyl]carbonyl}amino)-1H-benzimidazole-4-carboxamide,
(145) N-(3-chloro-2-methylphenyl)-2-{[2-(morpholin-4-yl)ethyl]amino}-6-({[2-(trifluoromethyl)phenyl]carbonyl}amino)-1H-benzimidazole-4-carboxamide,
(146) N-(3-chloro-2-methylphenyl)-2-{[2-(dimethylamino)ethyl]amino}-6-({[2-(trifluoromethyl)phenyl]carbonyl}amino)-1H-benzimidazole-4-carboxamide,
(147) N-(3-chloro-2-methylphenyl)-2-(3-hydroxyazetidin-1-yl)-6-({[2-(trifluoromethyl)phenyl]carbonyl}amino)-1H-benzimidazole-4-carboxamide,
(148) N-(3-chloro-2-methylphenyl)-2-[(3S)-3-(dimethylamino)pyrrolidin-1-yl]-6-({[2-(trifluoromethyl)phenyl]carbonyl}amino)-1H-benzimidazole-4-carboxamide,
(149) N-(3-chloro-2-methylphenyl)-2-[(3S)-3-hydroxypyrrolidin-1-yl]-6-({[2-(trifluoromethyl)phenyl]carbonyl}amino)-1H-benzimidazole-4-carboxamide,
(150) N-(3-chloro-2-methylphenyl)-2-{[2-(diethylamino)ethyl]amino}-6-({[2-(trifluoromethyl)phenyl]carbonyl}amino)-1H-benzimidazole-4-carboxamide,
(151) N-(3-chloro-2-methylphenyl)-2-{[2-(pyrrolidin-1-yl)ethyl]amino}-6-({[2-(trifluoromethyl)phenyl]carbonyl} amino)-1H-benzimidazole-4-carboxamide,
(152) N-(3-chloro-2-methylphenyl)-2-{[3-(dimethylamino)propyl]amino}-6-({[2-(trifluoromethyl)phenyl]carbonyl}amino)-1H-benzimidazole-4-carboxamide,
(153) N-(3-chloro-2-methylphenyl)-2-{[3-(dimethylamino)-2,2-dimethylpropyl]amino}-6-({[2-(trifluoromethyl)phenyl]carbonyl}amino)-1H-benzimidazole-4-carboxamide,
(154) N-(3-chloro-2-methylphenyl)-2-{[2-(dipropan-2-ylamino)ethyl]amino}-6-({[2-(trifluoromethyl)phenyl]carbonyl}amino)-1H-benzimidazole-4-carboxamide,
(155) N-(3-chloro-2-methylphenyl)-2-(morpholin-4-yl)-6-({[2-(trifluoromethyl)phenyl]carbonyl}amino)-1H-benzimidazole-4-carboxamide,
(156) 2-amino-N-(3-chloro-2-methylphenyl)-6-({[2-(trifluoromethyl)phenyl]carbonyl}amino)-1H-benzimidazole-4-carboxamide,
(157)N-(3-chloro-2-methylphenyl)-2-[(3-hydroxy-2,2-dimethylpropyl)amino]-6-({[2-(trifluoromethyl)phenyl]carbonyl}amino)-1H-benzimidazole-4-carboxamide,
(158) N-(3-chloro-2-methylphenyl)-2-{[(3-methyloxetan-3-yl)methyl]amino}-6-({[2-(trifluoromethyl)phenyl]carbonyl}amino)-1H-benzimidazole-4-carboxamide,
(159) tert-butyl N-{4-[(3-chloro-2-methylphenyl)carbamoyl]-6-({[2-(trifluoromethyl)phenyl]carbonyl}amino)-1H-benzimidazol-2-yl}glycinate,
(160) N-{4-[(3-chloro-2-methylphenyl)carbamoyl]-6-({[2-(trifluoromethyl)phenyl]carbonyl}amino)-1H-benzimidazol-2-yl}glycine,
(161) N-(3-chloro-2-methylphenyl)-2-[(3-hydroxy-2,2-dimethylpropyl)amino]-1-methyl-6-({[2-(trifluoromethyl)phenyl]carbonyl}amino)-1H-benzimidazole-4-carboxamide,
(162) N-(3-chloro-2-methylphenyl)-2-[(3-methoxy-2,2-dimethylpropyl)amino]-6-({[2-(trifluoromethyl)phenyl]carbonyl}amino)-1H-benzimidazole-4-carboxamide,
(163) N-(3-chloro-2-methylphenyl)-2-(pyrrolidin-1-yl)-6-({[2-(trifluoromethyl)phenyl]carbonyl}amino)-1H-benzimidazole-4-carboxamide,
(164) 2-(azetidin-1-yl)-N-(3-chloro-2-methylphenyl)-6-({[2-(trifluoromethyl)phenyl]carbonyl}amino)-1H-benzimidazole-4-carboxamide,
(165) N-(3-chloro-2-methylphenyl)-2-(3-methoxyazetidin-1-yl)-6-({[2-(trifluoromethyl)phenyl] carbonyl}amino)-1H-benzimidazole-4-carboxamide,
(166) N-(3-chloro-2-methylphenyl)-2-[(2-hydroxy-2-methylpropyl)amino]-6-({[2-(trifluoromethyl)phenyl]carbonyl}amino)-1H-benzimidazole-4-carboxamide,
(167) N-(3-chloro-2-methylphenyl)-2-{[(2S)-tetrahydrofuran-2-ylmethyl]amino}-6-({[2-(trifluoromethyl)phenyl]carbonyl}ammo)-1H-benzimidazole-4-carboxamide,
(168) N-(3-chloro-2-methylphenyl)-2-{[(2R)-tetrahydrofuran-2-ylmethyl]amino} -6-({[2-(trifluoromethyl)phenyl]carbonyl}amino)-1H-benzimidazole-4-carboxamide,
(169) N-(3-chloro-2-methylphenyl)-2-{[(2S)-1-hydroxy-3-methylbutan-2-yl]amino}-6-({[2-(trifluoromethyl)phenyl]carbonyl}amino)-1H-benzimidazole-4-carboxamide,
(170) N-(3-chloro-2-methylphenyl)-2- {[(2R)-1-hydroxy-3-methylbutan-2-yl]amino}-6-({[2-(trifluoromethyl)phenyl]carbonyl}amino)-1H-benzimidazole-4-carboxamide,
(171) N-(3-chloro-2-methylphenyl)-2-{[(2S)-1-hydroxy-3,3-dimethylbutan-2-yl]amino}-6-({[2-(trifluoromethyl)phenyl]carbonyl}amino)-1H-benzimidazole-4-carboxamide,
(172) N-(3-chloro-2-methylphenyl)-2-[(3-methoxy-2,2-dimethylpropyl)(methyl)amino]-6-({[2-(trifluoromethyl)phenyl]carbonyl}amino)-1H-benzimidazole-4-carboxamide,
(173) N-(3-chloro-2-methylphenyl)-2-[(3-methoxypropyl)amino]-6-({[2-(trifluoromethyl)phenyl]carbonyl}amino)-1H-benzimidazole-4-carboxamide,
(174) N-(3-chloro-2-methylphenyl)-2-{[2-(propan-2-yloxy)ethyl]amino}-6-({[2-(trifluoromethyl)phenyl]carbonyl}amino)-1H-benzimidazole-4-carboxamide,
(175) 2-[(2-tert-butoxyethyl)amino]-N-(3-chloro-2-methylphenyl)-6-({[2-(trifluoromethyl)phenyl]carbonyl}amino)-1H-benzimidazole-4-carboxamide,
(176) N-(3-chloro-2-methylphenyl)-2-[(2-methoxy-2-methylpropyl)amino]-6-({[2-(trifluoromethyl)phenyl]carbonyl}amino)-1H-benzimidazole-4-carboxamide,
(177) N-(3-chloro-2-methylphenyl)-2-{[2-(methylsulfanyl)ethyl]amino}-6-({[2-(trifluoromethyl)phenyl]carbonyl}amino)-1H-benzimidazole-4-carboxamide,
(178) N-(3-chloro-2-methylphenyl)-2-(methylsulfanyl)-6-({[2-(trifluoromethyl)phenyl]carbonyl} amino)-1H-benzimidazole-4-carboxamide,
(179) N-(3-chloro-2-methylphenyl)-2-(methylsulfonyl)-6-({[2-(trifluoromethyl)phenyl]carbonyl}amino)-1H-benzimidazole-4-carboxamide,
(180) N-(3-chloro-2-methylphenyl)-2-(methylsulfinyl)-6-({[2-(trifluoromethyl)phenyl]carbonyl} amino)-1H-benzimidazole-4-carboxamide,
(181) 6- {[(2-chloro-6-fluorophenyl)carbonyl]amino} -N-(3-chloro-2-methylphenyl)-2-(dimethylamino)-1H-benzimidazole-4-carboxamide,
(182) N-(3-chloro-2-methylphenyl)-6-{[(2,6-dichlorophenyl)carbonyl]amino}-2-(dimethylamino)-1H-benzimidazole-4-carboxamide,
(183) N-(3-chloro-2-methylphenyl)-6-{[(2,4-dichlorophenyl)carbonyl]amino}-2-(dimethylamino)-1H-benzimidazole-4-carboxamide,
(184) N-(3-chloro-2-methylphenyl)-6-{[(2,5-dichlorophenyl)carbonyl]amino}-2-(dimethylamino)-1H-benzimidazole-4-carboxamide,
(185) 6- {[(2-bromo-6-fluorophenyl)carbonyl]amino}-N-(3-chloro-2-methylphenyl)-2-(dimethylamino)-1H-benzimidazole-4-carboxamide,
(186) 6-{[(2-bromo-6-chlorophenyl)carbonyl]amino}-N-(3-chloro-2-methylphenyl)-2-(dimethylamino)-1H-benzimidazole-4-carboxamide,
(187) 6-({[2-chloro-5-(cyclopropylethynyl)phenyl]carbonyl}amino)-N-(3-chloro-2-methylphenyl)-2-(dimethylamino)-1H-benzimidazole-4-carboxamide,
(188) N-(3-chloro-2-methylphenyl)-6- {[(2,5-dichlorophenyl)carbonyl]amino} -2-[(3-hydroxy-2,2-dimethylpropyl)amino]-1H-benzimidazole-4-carboxamide,
(189) N-(3-chloro-2-methylphenyl)-6-{[(2,5-dichlorophenyl)carbonyl]amino}-2-[(3-methoxy-2,2-dimethylpropyl)amino]-1H-benzimidazole-4-carboxamide,
(190) N-(3-chloro-2-methylphenyl)-6-{[(2,5-dichlorophenyl)carbonyl]amino}-2-[(2-hydroxy-2-methylpropyl)amino]-1H-benzimidazole-4-carboxamide,
(191) N-(3-chloro-2-methylphenyl)-6-{[(2,5-dichlorophenyl)carbonyl]amino1-2-[(2-methoxy-2-methylpropyl)amino]-1H-benzimidazole-4-carboxamide,
(192) N-(3-chloro-2-methylphenyl)-6-{[(2,5-dichlorophenyl)carbonyl]amino}-2-{[2-(propan-2-yloxy)ethyl]amino}-1H-benzimidazole-4-carboxamide,
(193) 6-{[(2-chloro-6-fluorophenyl)carbonyl]amino}-N-(3-chloro-2-methylphenyl)-2-{[2-(propan-2-yloxy)ethyl]amino}-1H-benzimidazole-4-carboxamide,
(194) 2-[(2-tert-butoxyethyl)amino]-6-{[(2-chloro-6-fluorophenyl)carbonyl]amino} -N-(3-chloro-2-methylphenyl)-1H-benzimidazole-4-carboxamide,
(195) 6-{[(2-chloro-6-fluorophenyl)carbonyl]amino}-N-(3-chloro-2-methylphenyl)-2-[(3-methoxy-2,2-dimethylpropyl)amino]-1H-benzimidazole-4-carboxamide,
(196) 6-{[(2-chloro-6-fluorophenyl)carbonyl]amino}-N-(3-chloro-2-methylphenyl)-2-[(2-methoxy-2-methylpropyl)amino]-1H-benzimidazole-4-carboxamide,
(197) 6-{[(2-chloro-6-fluorophenyl)carbonyl]amino}-N-(3-chloro-2-methylphenyl)-2- {[(2S)-tetrahydrofuran-2-ylmethyl]amino}-1H-benzimidazole-4-carboxamide,
(198) 6-{[(2-chloro-6-fluorophenyl)carbonyl]amino}-N-(3-chloro-2-methylphenyl)-2-{[(2R)-tetrahydrofuran-2-ylmethyl]amino}-1H-benzimidazole-4-carboxamide,
(199) 6-{[(2-chloro-6-fluorophenyl)carbonyl]amino}-N-(3-chloro-2-methylphenyl)-2-[(3-hydroxy-2,2-dimethylpropyl)amino]-1H-benzimidazole-4-carboxamide,
(200) 6-{[(2-chloro-6-fluorophenyl)carbonyl]amino}-N-(3-chloro-2-methylphenyl)-2-{[(2S)-1-hydroxy-3-methylbutan-2-yl]amino}-1H-benzimidazole-4-carboxamide,
(201) N-(3-chloro-4-methylphenyl)-2-(dimethylamino)-6-({[2-(trifluoromethyl)phenyl]carbonyl}amino)-1H-benzimidazole-4-carboxamide,
(202) N-(4-tert-butylphenyl)-2-(dimethylamino)-6-({[2-(trifluoromethyl)phenyl]carbonyl}amino)-1H-benzimidazole-4-carboxamide,
(203) N-(2,3-dihydro-1H-inden-5-yl)-2-(dimethylamino)-6-({[2-(trifluoromethyl)phenyl]carbonyl}amino)-1H-benzimidazole-4-carboxamide,
(204) 6-{[(2-chloro-6-fluorophenyl)carbonyl]amino}-N-(3-chloro-4-methylphenyl)-2-(dimethylamino)-1H-benzimidazole-4-carboxamide,
(205) N-(3-chloro-4-methylphenyl)-6-{[(2,6-dichlorophenyl)carbonyl]amino}-2-(dimethylamino)-1H-benzimidazole-4-carboxamide,
(206) N-(3-chloro-4-methylphenyl)-6-{[(2,5-dichlorophenyl)carbonyl]amino}-2-(dimethylamino)-1H-benzimidazole-4-carboxamide,
(207) N-(3-chloro-2-methylphenyl)-2-cyclopropyl-6-{[(2,5-dichlorophenyl)carbonyl]amino}-1H-benzimidazole-4-carboxamide,
(208) N-(3-chloro-4-methylphenyl)-2-cyclopropyl-6-{[(2,5-dichlorophenyl)carbonyl]amino}-1H-benzimidazole-4-carboxamide,
(209) N-(3-chloro-2-methylphenyl)-6-{[(2,5-dichlorophenyl)carbonyl]amino}-2-(1-methylcyclopropyl)-1H-benzimidazole-4-carboxamide,
(210) N-(3-chloro-4-methylphenyl)-6-{[(2,5-dichlorophenyl)carbonyl]amino}-2-(1-methylcyclopropyl)-1H-benzimidazole-4-carboxamide,
(211) N-(3-chloro-2-methylphenyl)-2-(methoxymethyl)-6-({[2-(methylsulfonyl)phenyl]carbonyl}amino)-1H-benzimidazole-4-carboxamide,
(212) N-(3-chloro-4-methylphenyl)-6-{[(2,5-dichlorophenyl)carbonyl]amino}-2-(2-methoxyethyl)-1H-benzimidazole-4-carboxamide,
(213) 2-(methoxymethyl)-N-phenyl-6-({[2-(trifluoromethyl)phenyl]carbonyl}amino)-1H-benzimidazole-4-carboxamide,
(214) 2-(methoxymethyl)-N-propyl-6-({[2-(trifluoromethyl)phenyl]carbonyl}amino)-1H-benzimidazole-4-carboxamide,
(215) 2-(methoxymethyl)-N-(pyridin-3-yl)-6-({[2-(trifluoromethyl)phenyl]carbonyl}amino)-1H-benzimidazole-4-carboxamide,
(216) N-benzyl-2-(methoxymethyl)-6-({[2-(trifluoromethyl)phenyl]carbonyl}amino)-1H-benzimidazole-4-carboxamide,
(217) N-(cyclohexylmethyl)-2-(methoxymethyl)-6-({[2-(trifluoromethyl)phenyl]carbonyl}amino)-1H-benzimidazole-4-carboxamide,
(218) 2-(methoxymethyl)-N-(naphthalen-1-yl)-6-({[2-(trifluoromethyl)phenyl]carbonyl}amino)-1H-benzimidazole-4-carboxamide,
(219) 2-(methoxymethyl)-N-(thiophen-3-yl)-6-({[2-(trifluoromethyl)phenyl]carbonyl}amino)-1H-benzimidazole-4-carboxamide,
(220) N-(2,1,3-benzothiadiazol-4-yl)-2-(methoxymethyl)-6-({[2-(trifluoromethyl)phenyl]carbonyl}amino)-1H-benzimidazole-4-carboxamide,
(221) N-(1,1-dioxido-1-benzothiophen-6-yl)-2-(methoxymethyl)-6-({[2-(trifluoromethyl)phenyl]carbonyl}amino)-1H-benzimidazole-4-carboxamide,
(222) 2-(methoxymethyl)-N-(thiophen-2-ylmethyl)-6-({[2-(trifluoromethyl)phenyl]carbonyl}amino)-1H-benzimidazole-4-carboxamide,
(223) N-(1H-indol-5-yl)-2-(methoxymethyl)-6-({[2-(trifluoromethyl)phenyl]carbonyl}amino)-1H-benzimidazole-4-carboxamide,
(224) N-(1,3-benzothiazol-2-yl)-2-(methoxymethyl)-6-({[2-(trifluoromethyl)phenyl]carbonyl}amino)-1H-benzimidazole-4-carboxamide,
(225) N-(2,2-dimethylpropyl)-2-(methoxymethyl)-6-({[2-(trifluoromethyl)phenyl]carbonyl}amino)-1H-benzimidazole-4-carboxamide,
(226) 2-(methoxymethyl)-N-(thiophen-2-yl)-6-({[2-(trifluoromethyl)phenyl]carbonyl} amino)-1H-benzimidazole-4-carboxamide,
(227) N-(5-chloro-1,3-benzoxazol-2-yl)-2-(methoxymethyl)-6-({[2-(trifluoromethyl)phenyl]carbonyl} amino)-1H-benzimidazole-4-carboxamide,
(228) N-(2-benzylphenyl)-2-(methoxymethyl)-6-({[2-(trifluoromethyl)plienyl]carbonyl}amino)-1H-benzimidazole-4-carboxamide,
(229) 2-(methoxymethyl)-N-(quinolin-8-yl)-6-({[2-(trifluoromethyl)phenyl]carbonyl} amino)-1H-benzimidazole-4-carboxamide,
(230) N-(cycloheptylmethyl)-2-(methoxymethyl)-6-({[2-(trifluoromethyl)phenyl]carbonyl}amino)-1H-benzimidazole-4-carboxamide,
(231) N-(1,3-benzoxazol-2-yl)-2-(methoxymethyl)-6-({[2-(trifluoromethyl)phenyl]carbonyl}amino)-1H-benzimidazole-4-carboxamide,
(232) N-(6-chloro-1,3-benzoxazol-2-yl)-2-(methoxymethyl)-6-({[2-(trifluoromethyl)phenyl]carbonyl}amino)-1H-benzimidazole-4-carboxamide,
(233) N-[3 -chloro-2-(hydroxymethyl)phenyl]-2-(methoxymethyl)-6-({[2-(trifluoromethyl)phenyl]carbonyl} amino)-1H-benzimidazole-4-carboxamide,
(234) N-(3-chloro-2-methylphenyl)-6-{[(3-fluoropyridin-2-yl)carbonyl]amino}-2-(methoxymethyl)-1H-benzimidazole-4-carboxamide,
(235) N-(3-chloro-2-methylphenyl)-6-{[(3-chloropyridin-4-yl)carbonyl]amino}-2-(methoxymethyl)-1H-benzimidazole-4-carboxamide,
(236) N-(3-chloro-2-methylphenyl)-6-{[(3,5-dichloropyridin-4-yl)carbonyl]amino}-2-(methoxymethyl)-1H-benzimidazole-4-carboxamide,
(237) 6- {[(5-butoxy-2-chlorophenyl)carbonyl]amino} -N-(3-chloro-2-methylphenyl)-2-(methoxymethyl)-1H-benzimidazole-4-carboxamide,
(238) 6-({[2-chloro-5-(2,2-difluoroethoxy)phenyl]carbonyl}amino)-N-(3-chloro-2-methylphenyl)-2-(methoxymethyl)-1H-benzimidazole-4-carboxamide, and
(239) N-(3-chloro-2-methylphenyl)-6-({[2-chloro-5-(4,4,4-trifluorobutoxy)phenyl]carbonyl}amino)-2-(methoxymethyl)-1H-benzimidazole-4-carboxamide,

or a tautomer of the compound, or a pharmaceutically acceptable salt thereof.

**9.** The prophylactic and/or therapeutic agent for cancer according to any one of claims 2 to 8, wherein the mPGES-1 inhibitor is a compound selected from the group consisting of:

N-(3-chloro-2-methylphenyl)-2-(methoxymethyl)-6-({[2-(trifluoromethyl)phenyl]carbonyl}amino)-1H-benzimidazole-4-carboxamide,
N-(3-chloro-2-methylphenyl)-2-(methoxymethyl)-6-({[2-(trifluoromethyl)phenyl]carbonyl}amino)-1H-benzimidazole-4-carboxamide hydrochloride,
N-(3-chloro-2-methylphenyl)-2-(methoxymethyl)-6-({[2-(trifluoromethyl)phenyl]carbonyl}amino)-1H-benzimidazole-4-carboxamide methanesulfonate,
N-(3-chloro-2-methylphenyl)-2-(methoxymethyl)-6-({[2-(trifluoromethyl)phenyl]carbonyl} amino)-1H-benzimidazole-4-carboxamide 4-methylbenzenesulfonate,
N-(3-chloro-2-methylphenyl)-2-(methoxymethyl)-6-({[2-(trifluoromethyl)phenyl]carbonyl}amino)-1H-benzimidazole-4-carboxamide sulfate,
N-(3-chloro-2-methylphenyl)-2-(1-methylcyclopropyl)-6-({[2-(trifluoromethyl)phenyl]carbonyl}amino)-1H-benzimidazole-4-carboxamide,
N-(3-chloro-2-methylphenyl)-2-(1-methylcyclopropyl)-6-({[2-(trifluoromethyl)phenyl] carbonyl} amino)-1H-benzimidazole-4-carboxamide hydrochloride,
N-(3-chloro-2-methylphenyl)-2-(1-methylcyclopropyl)-6-({[2-(trifluoromethyl)phenyl]carbonyl} amino)-1H-benzimidazole-4-carboxamide methanesulfonate,
N-(3-chloro-2-methylphenyl)-2-(1-methylcyclopropyl)-6-({[2-(trifluoromethyl)phenyl]carbonyl} amino)-1H-benzimidazole-4-carboxamide 4-methylbenzenesulfonate,
N-(3-chloro-2-methylphenyl)-2-(1-methylcyclopropyl)-6-({[2-(trifluoromethyl)phenyl]carbonyl} amino)-1H-benzimidazole-4-carboxamide sulfate,
N-(3-chloro-4-methylphenyl)-6-{[(2,5-dichlorophenyl)carbonyl]amino}-2-(dimethylamino)-1H-benzimidazole-4-carboxamide,
N-(3-chloro-4-methylphenyl)-6- { [(2,5-dichlorophenyl)carbonyl]amino} -2-(dimethylamino)-1H-benzimidazole-4-carboxamide hydrochloride,
N-(3-chloro-4-methylphenyl)-6-{[(2,5-dichlorophenyl)carbonyl]amino}-2-(dimethylamino)-1H-benzimidazole-4-carboxamide methanesulfonate,
N-(3-chloro-4-methylphenyl)-6- { [(2,5-dichlorophenyl)carbonyl]amino} -2-(dimethylamino)-1H-benzimidazole-4-carboxamide 4-methylbenzenesulfonate, and
N-(3-chloro-4-methylphenyl)-6-{[(2,5-dichlorophenyl)carbonyl]amino}-2-(dimethylamino)-1H-benzimidazole-4-carboxamide sulfate,
or a tautomer of the compound, or a pharmaceutically acceptable salt thereof.

**10.** The prophylactic and/or therapeutic agent for cancer according to any one of claims 1 to 9,
wherein the immune checkpoint inhibitor has a mode of action selected from the group consisting of adenosine A2A receptor antagonist, adenosine A2B receptor antagonist, anti-5'-nucleotidase (anti-NTSE, anti-CD73), anti-CD 134 (antibody OX40), anti-CD 154 (anti-CD40L), anti-CD223 (anti-LAG-3: Lymphocyte Activation Gene 3 Protein), anti-CD27, anti-CD276 antigen (anti-B7-H3), anti-CD70, anti-CTLA4: Cytotoxic T-Lymphocyte Protein 4 (anti-CD152), anti-DLL1: delta-Like Protein 1, anti-ENTPDl (anti-CD39), anti-ILDR2: Immunoglobulin-Like Domain-Containing Receptor 2 (anti-C1orf32), anti-PD-1, anti-PD-LI (anti-CD274), anti-PVRIG: Transmembrane protein PVRIG, anti-TGFβ2: Transforming Growth Factor beta-2, anti-TIM3: Hepatitis A Virus Cellular Receptor 2, anti-VISTA: V-Type Immunoglobulin Domain-Containing Suppressor of T-cell Activation, antibody VTCN1 (anti-B7-H4, anti-Ovr110), CD47/SIRPalpha interaction inhibitor (Tyrosine-Protein Phosphatase Non-receptor Type Substrate 1), tryptophan-2,3-dioxygenase: TDO inhibition, and tubulin polymerization inhibition.

**11.** The prophylactic and/or therapeutic agent for cancer according to any one of claims 1 to 9,
wherein the immune checkpoint inhibitor is at least one selected from the group consisting of a 5'-nucleotidase inhibitor, an adenosine A2A receptor antagonist, an adenosine A2B receptor antagonist, an anti-CD73 antibody, an anti-CTLA-4/OX40 bispecific antibody, a PEGylated Fab antibody fragment against the CD40 ligand, an anti-CD40L Fc-fusion protein, an anti-CD40 ligand-Tn3 fusion protein, an anti-CD40L antibody, an anti-LAG-3 antibody, an anti-CD27 antibody, an anti-B7-H3 antibody, an anti-CD70 antibody, an anti-CTLA-4 antibody, a CTLA-4 targeting probody, an anti-CTLA-4/LAG-3 bispecific antibody, an anti-PD-LI/CTLA-4 bispecific antibody, an anti-PD-1 antibody having anti-DLL1 action, an anti-CD39 antibody, an anti-ILDR2 antibody, an anti-PD-1 antibody, a fusion protein of B7-DC and an antibody Fc portion, an anti-PD-1/anti-LAG-3 dual-affinity re-targeting (DART) protein, an anti-PD-1/CTLA-4 bispecific antibody, an anti-PD-1/CTLA-4 bispecific antibody, an anti-PD-1/ICOS bispecific antibody, an anti-PD-1/TIM-3 bispecific antibody, an anti-PD-1/PD-L1 bispecific antibody, an anti-PD-LI antibody, a protease-activated anti-PD-L1 antibody prodrug, an anti-PD-L1/CD137 bispecific antibody, an anti-LAG-3/PD-L1 bispecific antibody, an anti-PD-LI/TIM3 bispecific antibody, DuoBody-PD-L1x4-1BB (GEN-1046), an anti-PVRIG antibody, a bifunctional anti-PD-L1/TGFβ2 Trap fusion protein, an anti-TIM-3 antibody, a fully human Fc-engineered IgG1κ antibody targeting co-inhibitory receptor T-cell immunoglobulin and mucin, an anti-VISTA antibody, an anti-B7-H4 antibody, an anti-SIRPa antibody, a drug-bound antibody, an agonist redirected checkpoint (ARC) fusion protein consisting of the extracellular domains of human programmed cell death 1 (PD-1; PDCD1; CD279) and tumor necrosis factor ligand superfamily member 4 (TNFSF4; OX40 ligand; OX40L; CD252), linked by a central Fc domain (PD1-Fc-OX40L), an IDO-1 inhibitor a PD-L1 inhibitor, a PD-L1/PD-L2/VISTA antagonist, and a JAK2/STAT3 inhibitor.

**12.** The prophylactic and/or therapeutic agent for cancer according to any one of claims 1 to 9,
wherein the cancer is leukemia, malignant lymphoma, multiple myeloma, myelodysplastic syndrome, head and neck cancer, esophageal cancer, esophageal adenocarcinoma, gastric cancer, duodenal cancer, colorectal cancer, colon cancer, rectal cancer, liver cancer, gall bladder/bile duct cancer, biliary tract cancer, pancreatic cancer, thyroid cancer, lung cancer, breast cancer, ovarian cancer, cervical cancer, uterine body cancer, endometrial cancer, vaginal cancer, vulvar cancer, renal cancer, renal pelvis/urinary tract cancer, renal pelvic/ureteral cancer, urothelial cancer, penal cancer, prostate cancer, testicular tumor, bone/soft tissue sarcoma, malignant bone tumor, skin cancer, thymoma, mesothelioma, or cancer of unknown primary.

**13.** A prophylactic and/or therapeutic agent for cancer, containing an mPGES-1 inhibitor and an immune checkpoint inhibitor as active ingredients.

**14.** A pharmaceutical composition for prevention and/or treatment of cancer, the pharmaceutical composition containing an mPGES-1 inhibitor and a pharmaceutically acceptable carrier and for use in combination with an immune checkpoint inhibitor.

**15.** A pharmaceutical composition for prevention and/or treatment of cancer, the pharmaceutical composition containing an mPGES-1 inhibitor, an immune checkpoint inhibitor, and a pharmaceutically acceptable carrier.

**16.** An mPGES-1 inhibitor for use in combination with an immune checkpoint inhibitor for prevention and/or treatment of cancer.

**17.** Use of an mPGES-1 inhibitor for the manufacture of a medicine for use in combination with an immune checkpoint inhibitor in prevention and/or treatment of cancer.

**18.** A method for preventing and/or treating cancer, the method comprising administering an mPGES-1 inhibitor and an immune checkpoint inhibitor in combination to a subject in need of the combination.

Fig. 1

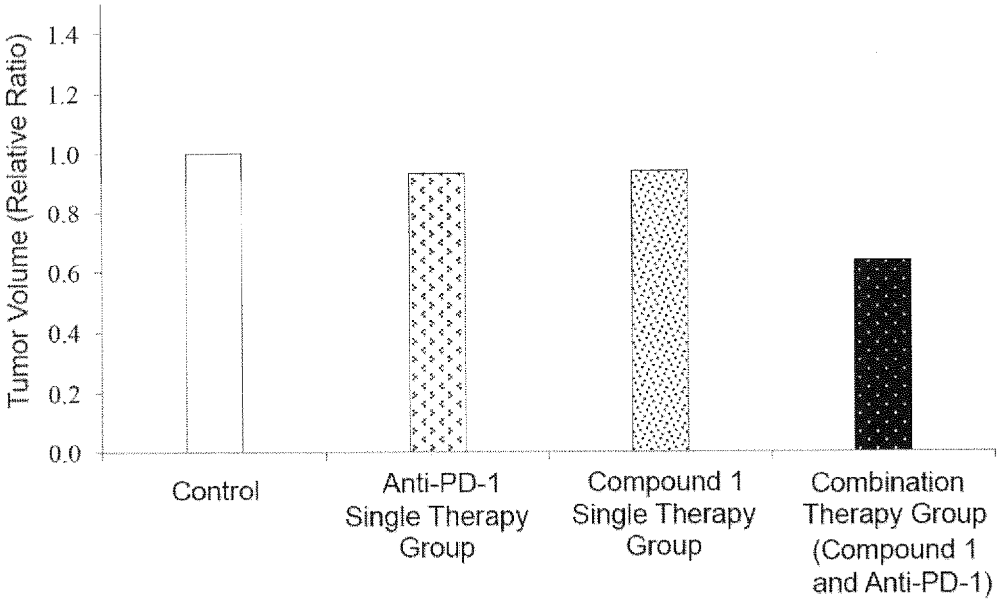
Tumor Volume (Relative Ratio)
1.4
1.2
1.0
0.8
0.6
0.4
0.2
0.0
Control
Anti-PD-1 Single Therapy Group
Compound 1 Single Therapy Group
Combination Therapy Group (Compound 1 and Anti-PD-1)

Fig. 2

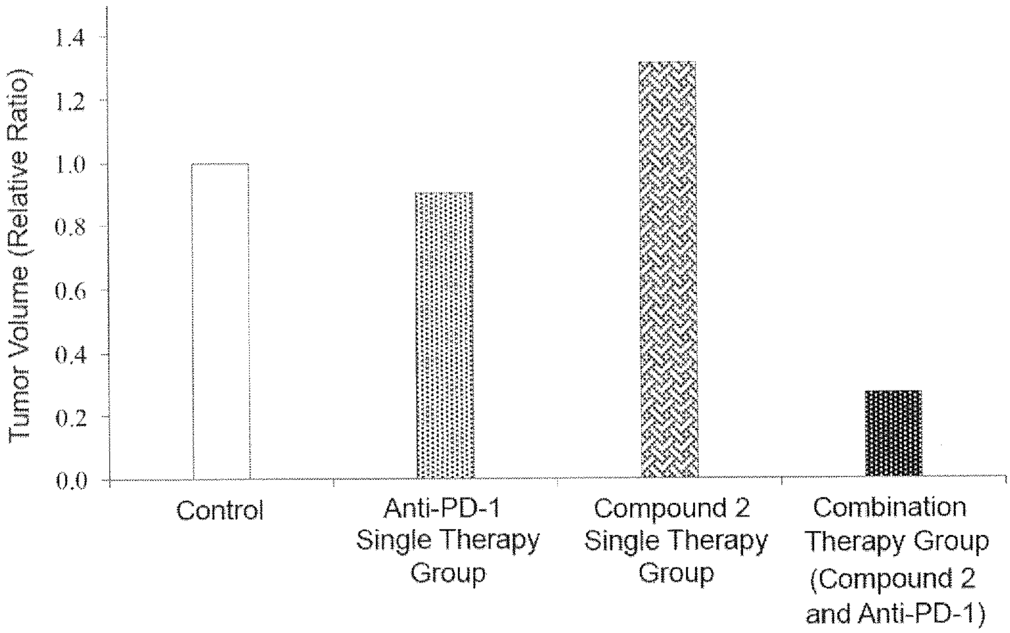
Tumor Volume (Relative Ratio)
1.4
1.2
1.0
0.8
0.6
0.4
0.2
0.0
Control
Anti-PD-1 Single Therapy Group
Compound 2 Single Therapy Group
Combination Therapy Group (Compound 2 and Anti-PD-1)

Fig. 3

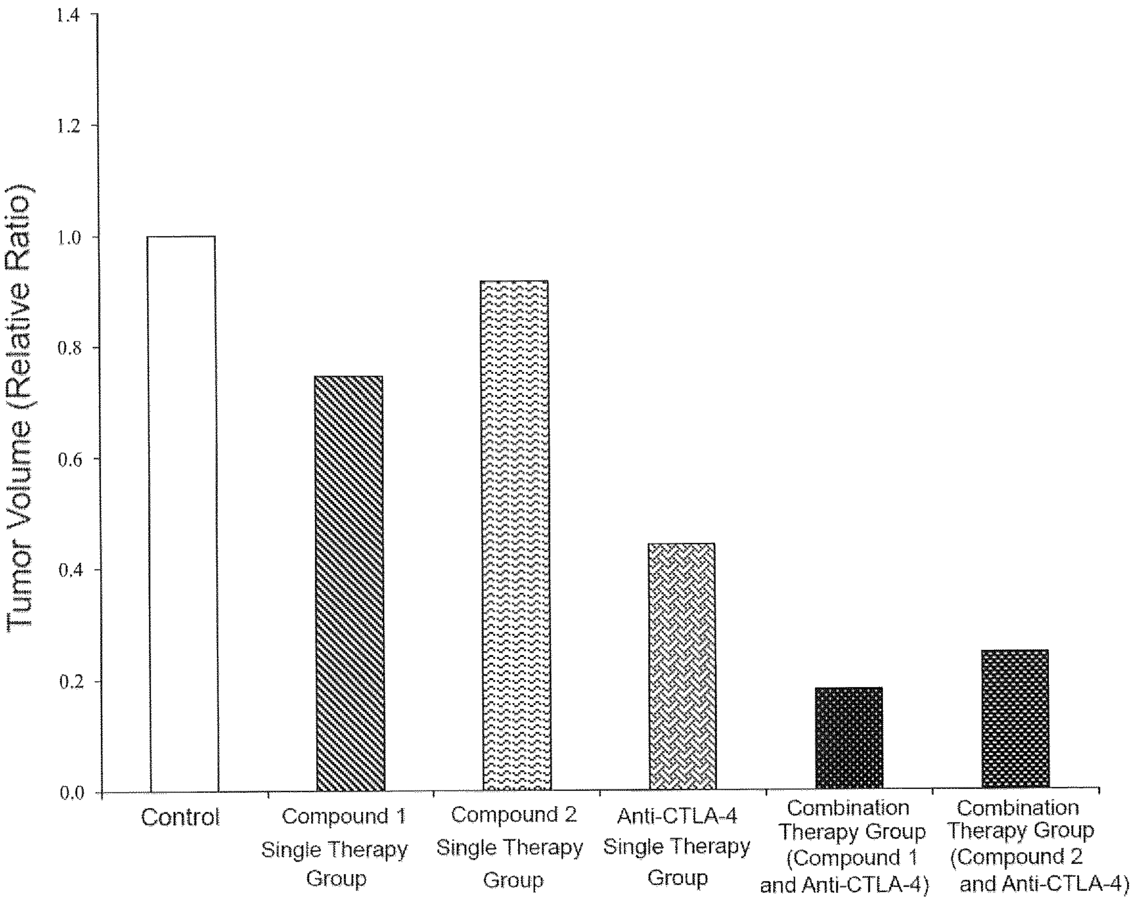
1.4
1.2
1.0
0.8
0.6
0.4
0.2
0.0
Tumor Volume (Relative Ratio)
Control
Compound 1 Single Therapy Group
Compound 2 Single Therapy Group
Anti-CTLA-4 Single Therapy Group
Combination Therapy Group (Compound 1 and Anti-CTLA-4)
Combination Therapy Group (Compound 2 and Anti-CTLA-4)

**INTERNATIONAL SEARCH REPORT**

International application No.
PCT/JP2020/048480

A. CLASSIFICATION OF SUBJECT MATTER
A61K 39/395(2006.01)i; A61K 45/06(2006.01)i; A61P 35/00(2006.01)i; A61P 43/00(2006.01)i; A61K 31/4184(2006.01)i
FI: A61K45/06; A61P43/00 121; A61P35/00; A61K39/395 D; A61K39/395 N; A61K31/4184

According to International Patent Classification (IPC) or to both national classification and IPC

B. FIELDS SEARCHED

Minimum documentation searched (classification system followed by classification symbols)
A61K39/395; A61K45/06; A61P35/00; A61P43/00; A61K31/4184

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII);
CAplus/REGISTRY/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| Y | PRIMA, V. et al., "COX2/mPGES1/PGE2 pathway regulates PD-L1 expression in tumor-associated macrophages and myeloid-derived suppressor cells.", Proceedings of the National Academy of Sciences of the United States of America, 2017, vol. 114 no. 5, pp. 1117-1122, ISSN: 0027-8 in particular, abstract, page 1121, right column, paragraph [0002] | 1-18 |
| Y | KAMEI, D. et al., "Potential Role of Microsomal Prostaglandin E Synthase-1 in Tumorigenesis.", Journal of Biological Chemistry, 2003, vol. 278, no. 21, pp. 19396-19405, DOI 10.1074/jbc.M213290200 in particular, abstract, etc. | 1-18 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 February 2021 (17.02.2021) | 02 March 2021 (02.03.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2020/048480

C (Continuation). DOCUMENTS CONSIDERED TO BE RELEVANT

| Category* | Citation of document, with indication, where appropriate, of the relevant passages | Relevant to claim No. |
|---|---|---|
| Y | SASAKI, Y. et al., "Microsomal prostaglandin E synthase-1 is involved in multiple steps of colon carcinogenesis.", Oncogene, 2012, vol. 31, pp. 2943-2952, ISSN: 0950-9232 in particular, abstract, fig. 1-3, 5 | 1-18 |
| Y | NAKANISHI, M. et al., "Genetic Deletion of mPGES-1 Suppresses Intestinal Tumorigenesis.", Cancer Research, 2008, vol. 68 no. 9, pp. 3251-3259, DOI:10.1158/0008-5472. in particular, abstract, fig. 1 | 1-18 |
| Y | WO 2013/024898 A1 (NIPPON SHINYAKU CO., LTD.) 21 February 2013 (2013-02-21) in particular, examples | 2-12 |

Form PCT/ISA/210 (continuation of second sheet) (January 2015)

**INTERNATIONAL SEARCH REPORT**
Information on patent family members

International application No.
PCT/JP2020/048480

| Patent Documents referred in the Report | Publication Date | Patent Family | Publication Date |
|---|---|---|---|
| WO 2013/024898 A1 | 21 Feb. 2013 | US 2014/0221339 A1 (examples)<br>EP 2746265 A1<br>CN 103857657 A<br>KR 10-2014-0054193 A<br>AU 2012295805 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013024898 A


### Non-patent literature cited in the description


- *J. Biol. Chem.,* 2003, vol. 278 (21), 19396-19405
- *Oncogene,* 2012, vol. 31 (24), 2943-2952
- *Cancer Res.,* 2008, vol. 68 (9), 3251-3259
- *Front Immunol.,* 27 July 2018, vol. 9, 1739
- *Cancer Res.,* 2013, vol. 73 (12), 3591-603
- *Cancer Res,* 2013, vol. 73 (12), 3591-603